(19) Europäisches Patentamt
European Patent Office
Office européen des brevets

(11) EP 0 707 059 B1

(12) **EUROPEAN PATENT SPECIFICATION**

(45) Date of publication and mention of the grant of the patent:
**08.09.2004 Bulletin 2004/37**

(21) Application number: **95116186.8**

(22) Date of filing: **13.10.1995**

(51) Int Cl.[7]: **C11D 3/00**, C07C 237/06, C11D 1/825, C11D 1/835, C07C 211/63, C07C 219/06, C07C 231/12, C07C 233/38, C07C 209/04, C07C 213/08, C07C 233/36

(54) **Liquid softener composition**

Flüssige Weichspülerzusammensetzung

Composition adoucissante liquide

(84) Designated Contracting States:
**DE ES FR GB IT**

(30) Priority: **14.10.1994 JP 24911594**
**25.10.1994 JP 26008094**

(43) Date of publication of application:
**17.04.1996 Bulletin 1996/16**

(60) Divisional application:
**04009337.9 / 1 445 303**

(73) Proprietor: **KAO CORPORATION**
**Chuo-ku, Tokyo (JP)**

(72) Inventors:
- **Sakata, Yushi**
  **Wakayama-shi, Wakayama (JP)**
- **Inokoshi, Junichi**
  **Wakayama-shi, Wakayama (JP)**
- **Nishimoto, Uichiro**
  **Wakayama-shi, Wakayama (JP)**
- **Kato, Tohru**
  **Wakayama-shi, Wakayama (JP)**
- **Bermejo Osés, Ma. José,**
  **c/o Kao Corporation S.A.**
  **E-08210 Barberà del Vallès, Barcelona (ES)**
- **Okabe, Kazuhiko, c/o Kao Corporation S.A.**
  **E-08210 Barberà del Vallès, Barcelona (ES)**
- **Sotoya, Kohshiro**
  **Naga-gun, Wakayama (JP)**

(74) Representative:
**Hansen, Bernd, Dr. Dipl.-Chem. et al**
**Hoffmann Eitle,**
**Patent- und Rechtsanwälte,**
**Arabellastrasse 4**
**81925 München (DE)**

(56) References cited:

**EP-A- 0 008 839**
**EP-A- 0 023 334**
**EP-A- 0 025 165**
**EP-A- 0 159 919**
**EP-A- 0 586 323**
**EP-A- 0 643 128**
**WO-A-94/06900**
**WO-A-94/21592**
**WO-A-94/21593**
**DE-A- 4 135 115**
**GB-A- 1 453 385**
**GB-A- 2 202 244**
**US-A- 5 282 983**

- **PATENT ABSTRACTS OF JAPAN vol. 013, no. 171 (C-588), 24 April 1989 (1989-04-24) & JP 64 001794 A (KAO CORP), 6 January 1989 (1989-01-06)**
- **DATABASE CHEMABS [Online] CHEMICAL ABSTRACT SERVICE, COLUMBUS, OHIO, US; STN, CAPLUS AN 1996:422308, XP002160304 & JP 08 104668 A (KAO CORP.) 23 April 1996 (1996-04-23)**
- **DATABASE CHEMABS [Online] CHEMICAL ABSTRACT SERVICE, COLUMBUS, OHIO, US; STN, CAPLUS AN 1996:337882, XP002160305 & JP 08 059573 A (KAO CORP.) 5 March 1996 (1996-03-05)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 707 059 B1

## Description

### Background of the Invention

### Field of the Invention

The present invention relates to a liquid softener composition for clothes which is applicable to a rinsing bath for the washing and can impart excellent softness, antistatic properties and water absorption properties to various textiles.

### Prior Art

Clothes have a tendency to be stiffened owing to the washing-away of textile auxiliaries or the degradation of fiber itself during repeated wear and washing to result in uncomfortable hand. Therefore, softeners which can impart softness and antistatic properties to textile have recently been used in many households.

Such a softener lowers the frictional coefficient of fiber surface by the lubricating effect due to the lipophilic moiety of the base material molecule of the softener adsorbed on the fiber surface to thereby exhibit a softening effect. When a textile is treated with a softener containing a base material having an excellent softening effect, however, the resulting textile cannot avoid becoming greasy to the touch. In particular, when underwear which comes into direct contact with the skin (such as cotton knit underwear or nylon tricot slip) or a towel which comes into direct contact with the skin and the hand of which the user is sensitive to was treated with such. a softener, the resulting underwear or towel had a problem of being greasy to the touch, though the greasiness of the textile treated with the softener varies depending upon the kind of fiber, the method of knitting and so forth. Further, when the softener of the prior art was used in the treatment of clothes in a higher concentration, a higher softening effect was attained but the greasiness of the resulting clothes was more significant. Accordingly, it was believed with respect to the softener composition of the prior art that there was a correlation between softening effect and greasiness.

Under these circumstances, there have been proposed a softener for clothes comprising a neutralization or quaternization product of an amine compound having ester and amide groups in its molecule and an adduct of fatty acid derivative with alkylene oxide (see Japanese Patent Laid-Open No. 57632/1994), a softener usable in rising after washing which comprises an alkoxylated natural fat or oil (see Japanese Patent Laid-Open No. 10263/1994) and so forth. When cloth is treated with these softener compositions, no excellent softening effect is found in some cases, while excellent softening effect is found with undesirable greasy touch in the other cases. No satisfactory softener has been developed as yet.

### Disclosure of the Invention

The present invention aims at providing a liquid softener composition which is applicable to a rinsing bath for the washing and can impart excellent softness to cloth without making the cloth greasy to the touch.

The inventors of the present invention have intensively studied to attain the above aim, and have found as a result of the studies that the above aim can be attained by combining severely selected components. The present invention has been accomplished on the basis of this finding.

The present invention provides a liquid softener composition comprising the following softener base material (1) or (2) and water:

[softener base material (1)]

a mixture comprising a compound represented by the following general formula (I) (hereinafter referred to as "compound (I)"), a compound represented by the following general formula (II) (hereinafter referred to as "compound (II)"), a compound represented by the following general formula (III) (hereinafter referred to as "compound (III)") and a compound represented by the following general formula (IV) (hereinafter referred to as "compound (IV)"), provided that

the weight ratio of the compound (I) to the sum total of the compounds (I), (II), (III) and (IV) ranges from 0.040 to 0.327,

the weight ratio of the compound (II) to the sum total of the compounds (I), (II), (III) and (IV) ranges from 0.298 to 0.469,

the weight ratio of the compound (III) to the sum total of the compounds (I), (II), (III) and (IV) ranges from 0.173 to 0.661, and

the weight ratio of the compound (IV) to the total sum of the compounds (I), (II), (III) and (IV) ranges from

0.001 to 0.077,

and that the mixture comprising the compounds (I), (II), (III) and (IV) has a Griffin's HLB value ranging from 5 to 12: ..

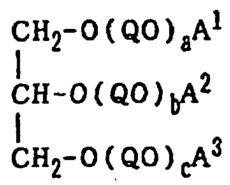

(I)

[wherein

$A^1$, $A^2$ and $A^3$: each an RCO group or a hydrogen atom with the proviso that one of $A^1$, $A^2$ and $A^3$ is an RCO group and the others thereof are each a hydrogen atom, wherein R represents a linear or branched alkyl or alkenyl group having 7 to 23 carbon atoms,

Q: an alkylene group having 2 or 3 carbon atoms or ar alkylene group mixture comprising one having two carbon atoms and one having three carbon atoms, and

a, b and c: each a number of 0 or above, provided that the sum of a, b and c is 4 to 18 on the average],

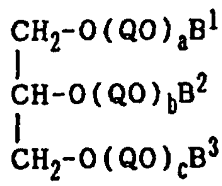

(II)

[wherein

$B^1$, $B^2$ and $B^3$: each an RCO group or a hydrogen atom with the proviso that two of $B^1$, $B^2$ and $B^3$ are each an RCO group and the other thereof is a hydrogen atom, wherein R group is as defined above, and

Q, a, b and c: each as defined above],

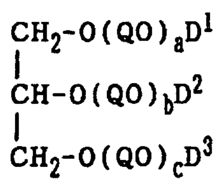

(III)

[wherein

$D^1$, $D^2$ and $D^3$: each an RCO group wherein R is as defined above, and

Q, a, b and c: each as defined above], and

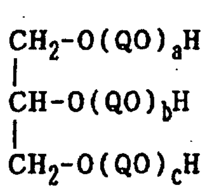

(IV)

[wherein

Q, a, b and c: each as defined above]

and

[softener base material (2)]

a mixture comprising the following components (A) and (B) wherein the weight ratio of the component (A) to the component (B) ranges from 2/1 to 1/9:

<component (A)>

a mixture comprising a compound represented by the following general formula (V) (hereinafter referred to as "compound (V)"), a compound represented by the following general formula (VI) (hereinafter referred to as "compound (VI)"), a compound represented by the following general formula (VII) (hereinafter referred to as "compound (VII)") and a compound represented by the following general formula (VIII) (hereinafter referred to as "compound (VIII)"), provided that

the weight ratio of the compound (V) to the total sum of the compounds (V), (VI), (VII) and (VIII) ranges from 0.040 to 0.527,
the weight ratio of the compound (VI) to the total sum of the compounds (V), (VI), (VII) and (VIII) ranges from 0.133 to 0.469,
the weight ratio of the compound (VII) to the total sum : of the compounds (V), (VI), (VII) and (VIII) ranges from 0.013 to 0.661, and
the weight ratio of the compound (VIII) to the total sum of the compounds (V), (VI), (VII) and (VIII) ranges from 0.001 to 0.417,

and that the mixture comprising the compounds (V). (VI) (VII) and (VIII) has a Griffind's HLB value ranging from 5 to 15, except the mixtures falling under the category of the softener base material (1):

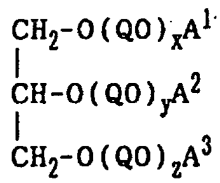

(V)

[wherein

$A^1$, $A^2$, $A^3$ and Q: each as defined above, and

x, y and z: each a number of 0 or above, provided that the sum of x, y and z is 1 to 50 on the average],

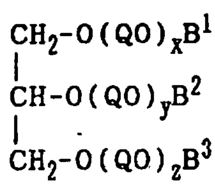

(VI)

[wherein

$B^1$, $B^2$, $B^3$, Q, x, y and z: each as defined above] ,

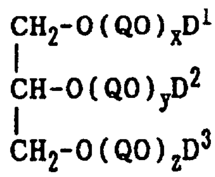

(VII)

[wherein

D1, D2, D3, Q, x, y and z: each as defined above] and

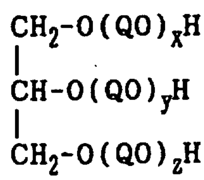

(VIII)

[wherein

Q, x, y and z: each as defined above], and

<component (B)>

at least one member selected from the following components (B-1) and (B-2):

<<component (B-1)>>

a compound represented by the following general formula (X):

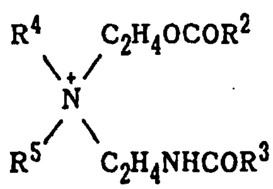

$X^-$ (X)

[wherein

$R^2$ and $R^3$: the same or different from each other a linear or branched alkyl or alkenyl group having 7 to 23 carbon atoms,

$R^4$ and $R^5$: each an alkyl group having 1 to 4 carbon atoms, and

$X^-$: a halide ion or $R^4$ $SO_4$ wherein $R^4$ is as defined above], and

<<component (B-2)>>

a compound represented by the following general formula (XI):

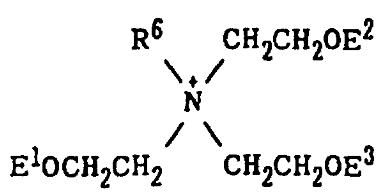

$Y^-$ (XI)

[wherein

$E^1$, $E^2$ and $E^3$: each an $R^7CO$ group or a hydrogen atom with the proviso that at least one of $E^1$, $E^2$ and $E^3$ is an $R^7CO$ group, wherein $R^7$ represents a linear or branched alkyl or alkenyl group having 7 to 23 carbon atoms,
$R^6$: an alkyl group having 1 to 4 carbon atoms, and
$Y^-$: a halide ion or $R^6SO_4$ wherein $R^6$ is as defined above].

Further, the present invention also provides a liquid softener composition comprising the above softener base material (1) and the component (B) described above as the constituent of the softener base material (2) wherein the weight ratio of the softener base material (1) to the component (B) ranges from 2/1 to 1/9.
The present invention will now be described in more detail.

Detailed Description of the Invention

[Softener base material (1)]

In the softener base material (1) comprising the compounds (I) to (IV), the mixing ratio must satisfy the requirement that the weight ratio of the compound (I) to the sum total of the compounds (I), (II), (III) and (IV) should range from 0.040 to 0.327, preferably 0.040 to 0.317, that of the compound (II) to the sum total of the compounds (I), (II), (III) and (IV) should range from 0.298 to 0.469, that of the compound (III) to the sum total of the compounds (I), (II), (III) and (IV) should range from 0.173 to 0.661, preferably 0.183 to 0.661 and that of the compound (IV) to the sum total of the compounds (I), (II), (III) and (IV) should range from 0.001 to 0.077, preferably 0.001 to 0.067.
When the mixing ratio satisfies the above requirement, the resulting softener composition will be good in stability. Although the reason for this phenomenon is not always apparent, it is presumed that the softener base material (1) having such a mixing ratio as described above may have a lowered crystallinity and therefore the resulting liquid softener composition may be inhibited from the deposition of the base material (1) to cause neither thickening nor gelation nor any other appearance change.
In each of the general formulae (I), (II), (III) and (IV), the sum of a, b and c is 4 to 18, preferably 4 to 12 on the average. When the sum of a, b and c fall within said range it is especially possible to prepare a stable softener composition and to attain the desirable softening effect.
It is essential that the Griffin's HLB value of the softener base material (1) is 5 to 12, preferably 5.6 to 12. When the HLB value falls within said range, it is in particular possible to prepare a stable softener composition and to attain a satisfactory softening effect
The Griffin's HLB value of a fatty acid ester of polyhydric alcohol is calculated by the following formula:

$$\text{Griffin's HLB} = 20 \times (1- S/A)$$

[wherein

S: saponification value (mg KOH/g) of the fatty acid ester of polyhydric alcohol, and
A: acid value (mg KOH/g) of the fatty acid constituting the fatty acid ester of polyhydric alochol].

In the general formulae (I) to (III), R group is, e.g., at least one alkyl group selected from the group consisting of alkyl resulting from tallow fatty acid, that resulting from hardened tallow fatty acid, that of palm stearic acid and that of hardened palm stearic acid.
The softener base material (1) can be prepared by, e.g., the following synthesis processes (i) to (iv).

Synthesis process (i)

The softener base material (1) can be prepared by esterifying glycerol with a fatty acid and conducting the addition reaction of the obtained ester with ethylene oxide and/or propylene oxide. In the addition thereof with both ethylene oxide and propylene oxide, it is preferable that the addition reaction with propylene oxide be followed by that with ethylene oxide, though the addition reaction with either of ethylene oxide and propylene oxide may precede or the addition reactions with ethylene oxide and propylene oxide may be conducted simultaneously. Further, it is preferable that the number of propylene oxide molecules added to one glycerol skeleton be 3 or below.
Although the above esterification according to the synthesis process (i) can be conducted without a catalyst, it may be conducted in the presence of an acid catalyst such as sulfuric acid, hydrochloric acid or p-toluenesulfonic acid. Examples of the fatty acid to be used in the esterification include caprinic acid, lauric acid, myristic acid, palmitic

acid, oleinic acid, stearic acid, isostearic acid, arachidic acid, behenic acid and fatty acids prepared from coconut oil, palm kernel oil, unhardened and hardened tallows, lard, palm oil, rape seed oil, fish oil, palm stearin and hardened palm stearin, which may be each used alone or as a mixture of two or more of them. Among these fatty acids, it is preferable to use coconut oil fatty acid, palm kernel oil fatty acid, palm oil fatty acid, unhardened or hardened tallow fatty acid, unhardened or hardened palm stearic acid or a mixture of two or more of them.

The addition reaction is conducted in the presence of NaOH, KOH, $NaOCH_3$, $KOCH_3$, an alkali metal salt of fatty acid or the like as catalyst. It is preferable that the amount of the catalyst to be added exceed the amount necessary for the addition reaction by the amount required for neutralizing the acid catalyst used for the above esterification.

Synthesis process (ii)

The softener base material (1) can be prepared by transesterifying a fatty acid ester with glycerol and conducting the addition reaction of the obtained ester with an alkylene oxide.

The catalyst to be used in the transesterification includes NaOH, KOH, $NaOCH_3$ and $KOCH_3$.

Examples of the fatty acid ester to be used in the synthesis process (ii) include esters of the fatty acids described in the section "Synthesis process (i)" with methanol, ethanol, propanol, butanol, ethylene glycol, glycerol, erythritol, pentaerythritol, xylitol, sorbitol and sorbitan.

Among these fatty acid esters, the following compounds are preferable:

$$R\text{-}COOCH_3, \qquad R\text{-}COOCH_2CH_3,$$

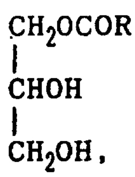 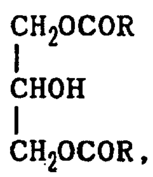 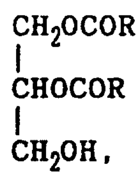 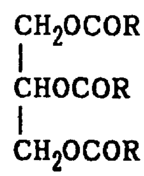

[wherein R is as defined above].

The addition reaction of the synthesis process (ii) is conducted in the presence of a catalyst such as NaOH, KOH, $NaOCH_3$, $KOCH_3$ or an alkali metal salt of fatty acid. The catalyst used in the preceding transesterification may be used as such in the addition reaction. The kind of alkylene oxide and the order of addition reaction are the same as described in the section "Synthesis process (i)".

Synthesis process (iii)

The softener base material (1) can be prepared by conducting the addition reaction of glycerol with an alkylene oxide and esterifying the obtained adduct with a fatty acid.

The addition reaction and esterification of the synthesis process (iii) may be conducted under the same conditions as those described in the section "Synthesis process (i)".

The fatty acid to be used in the esterification of the synthesis process (iii) may be selected from among those described in the section "Synthesis process (i)".

Synthesis process (iv)

The softener base material (1) can be prepared by conducting the addition reaction of glycerol with an alkylene oxide and transesterifying a fatty acid ester with the obtained adduct.

The addition reaction and transesterification of the synthesis process (iv) may be conducted under the same conditions as those described in the section "Synthesis process (ii)".

The fatty acid ester to be used in the transesterification of the synthesis process (iv) includes the following compounds:

$$R\text{-}COOCH_3, \qquad R\text{-}COOCH_2CH_3$$

[wherein R is as defined above].

Specific examples of the softener base material (1) include adducts of mixtures comprising glycerides of tallow fatty acid and glycerol with alkylene oxides, those of mixtures comprising glycerides of partially hardened tallow fatty acid and glycerol with alkylene oxides, those of mixtures comprising glycerides of palm stearic acid and glycerol with alkylene oxides, those of mixtures comprising glycerides of hardened palm stearic acid and glycerol with alkylene oxides, those of mixtures comprising lauric acid esters and glycerol with alkylene oxides, those of mixtures comprising glycerides of palm kernel oil fatty acid and glycerol with alkylene oxides, those of mixtures comprising glycerides of coconut oil fatty acid and glycerol with alkylene oxides, and those of mixtures comprising glycerides of palm oil fatty acid and glycerol with alkylene oxides. These adducts of alkylene oxides are preferably those of ethylene oxide.

[Softener base material (2)]

The softener base material (2) according to the present invention is a mixture comprising the above components (A) and (B). The weight ratio of the component (A) to the component (B) ranges from 2/1 to 1/9, preferably 1/1 to 1/9, still preferably 1/2 to 1/9. When the weight ratio falls within the mentioned range it is in particular possible to attain the desired effects according to the present invention.

<Component (A)>

The component (A) according to the present invention is a mixture comprising the above compounds (V), (VI), (VII) and (VIII), except the mixtures falling under the category of the softener base material (1). In the component (A) comprising the compounds (V) to (VIII), the mixing ratio must satisfy the requirement that the weight ratio of the compound (V) to the sum total of the compounds (V), (VI), (VII) and (VIII) should range from 0.040 to 0.527, preferably 0.047 to 0.346, still preferably 0.163 to 0.346, that of the compound (VI) to the sum total of the compounds (V), (VI), (VII) and (VIII) should range from 0.133 to 0.469, preferably 0.310 to 0.458, still preferably 0.404 to 0.458, that of the compound (VII) to the sum total of the compounds (V), (VI), (VII) and (VIII) should range from 0.013 to 0.661, preferably 0.154 to 0.641, still preferably 0.154 to 0.375, and that of the compound (VIII) to the sum total of the compounds (V), (VI), (VII) and (VIII) should range from 0.001 to 0.417, preferably 0.002 to 0.096, still preferably 0.017 to 0.096.

When the mixing ratio satisfies the above requirement, the resulting softener composition will be good in stability. Although the reason for this phenomenon is not always apparent, it is presumed that the component (A) having such a mixing ratio as described above may have a lowered crystallinity and therefore the resulting liquid softener composition may be inhibited from the deposition of the component (A) to cause neither thickening nor gelation nor any other appearance change.

In each of the general formulae (V), (VI), (VII) and (VIII), the sum of x, y and z is 1 to 50, preferably 3 to 50, still preferably 3 to 40 on the average. When the sum of x, y and z falls within this range particularly a stable softener composition will be prepared and the desirable softening effect according to the present invention will be attained.

The component (A) constituting the softener base material (2) must have a Griffin's HLB value ranging from 5 to 15, so that a stable softener composition may be prepared and a satisfactory softening effect may be attained.

In the general formulae (V) to (VII), R group is, e.g., at least one alkyl group selected from the group consisting of alkyl resulting from coconut oil fatty acid, that resulting from palm kernel oil fatty acid, that resulting from palm oil fatty acid, that resulting from palm stearic acid, that resulting hardened palm stearic acid, that resulting from tallow fatty acid and that resulting from hardened tallow fatty acid.

The component (A) constituting the softener base material (2) can be prepared by the same processes as those for preparing the softener base material (1).

In the general formulae (V), (VI), (VII) and (VIII), the alkylene oxide added is preferably ethylene oxide.

<Component (B)>

The component (B) according to the present invention is at least one member selected from the group consisting of the compounds (B-1) represented by the general formula (X) and the compounds (B-2) represented by the general formula (XI).

<<Component (B-1)>>

In the general formula (X), $X^-$ represents a halide ion (such as $Cl^-$, $Br^-$ or the like), $CH_3SO_4^-$, $C_2H_5SO_4^-$, $C_3H_7SO_4^-$ or the like.

The quaternary ammonium salt (X) to be used as the component (B-1) can be prepared according, e.g., the following reaction formula:

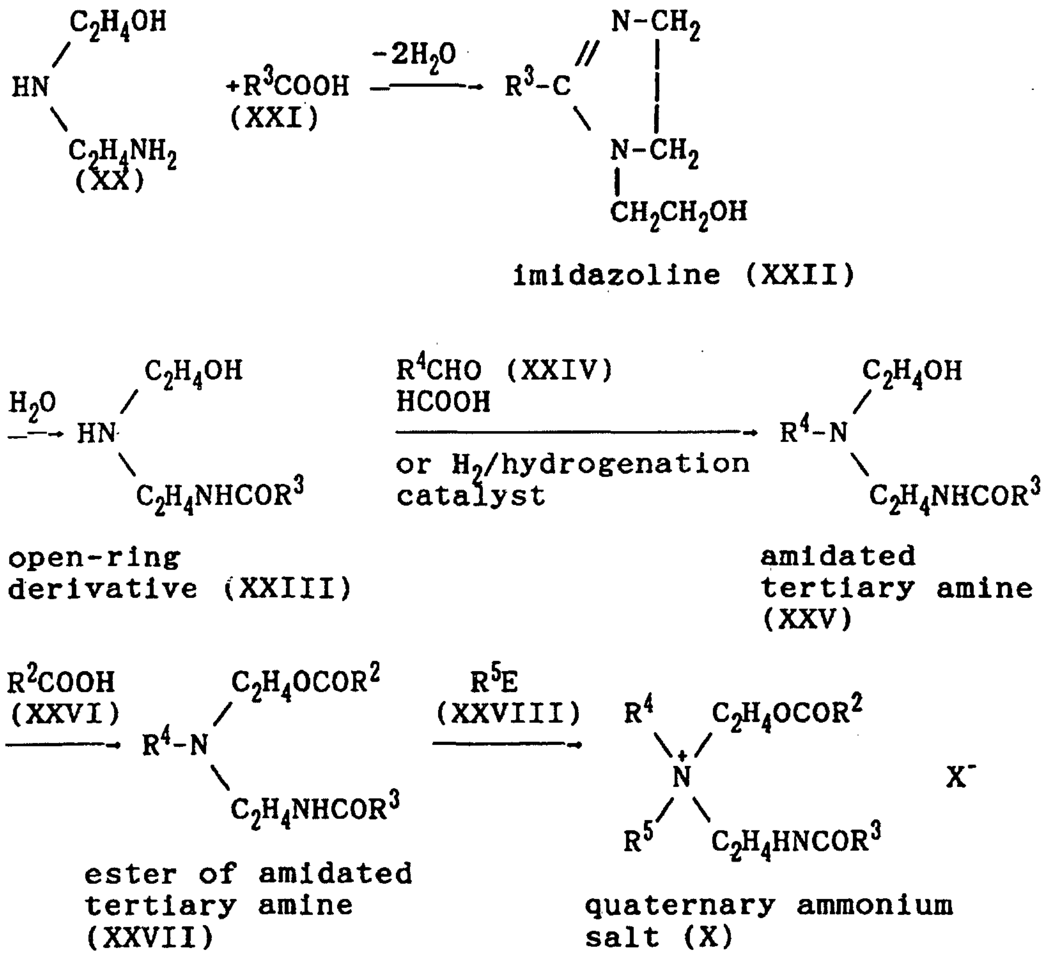


(XX)

imidazoline (XXII)

open-ring derivative (XXIII)

amidated tertiary amine (XXV)

ester of amidated tertiary amine (XXVII)

quaternary ammonium salt (X)

[wherein

| | |
|---|---|
| $R^2$, $R^3$, $R^4$, $R^5$ and $X^-$: | each as defined above, and |
| E: | a halogen atom or $R^5$-$SO_4^-$ wherein $R^5$ is as defined above]. |

Specifically, an imidazoline (XXII) is first prepared by the reaction of N-(2-aminoethyl)-ethanolamine (XX) with a fatty acid (XXI). The molar ratio of N-(2-aminoethyl)ethanolamine (XX) to the fatty acid (XXI) may range from 1.0 to 2.0., preferably 1.1 to 1.5. The reaction temperature may ranges from 150 to 250°C, preferably 180 to 230°C. It is preferable in order to enhance the dehydration efficiency that the reaction system be gradually vacuumized to about 5 Torr.

The fatty acid (XXI) to be used in the above reaction is generally one resulting from a natural fat or oil such as coconut oil, palm oil, tallow, rape seed oil or fish oil, or alternatively may be a synthetic fatty acid having 8 to 24 carbon atoms.

The imidazoline (XXII) thus prepared is hydrolyzed into an open-ring derivative (XXIII) in the presence of a catalyst such as NaOH or without a catalyst either in a mixed solvent such as water/ethanol or in water. In this hydrolysis, the amount of water used is 1 to 10 mol, preferably 2 to 5 mol per mol of the imidazoline (XXII), while ethanol is used in an amount of 0 to 50% by weight, preferably 10 to 30% by weight based on the sum total of the imidazoline (XXII) and water. Sodium hydroxide is used in an amount of 0 to 10% by mole, preferably 0 to 5% by mole based on the imidazoline (XXII).

The open-ring derivative (XXIII) thus prepared is converted into an amidated tertiary amine (XXV) either by subjecting the derivative (XXIII) to the Leukart reaction with an aldehyde (XXIV) such as formaldehyde or acetaldehyde under conventional conditions or by alkylating the derivative (XXIII) through reduction (i.e., by reacting the derivative (XXIII) with an aldehyde (XXIV) and hydrogenating the obtained product in the presence of a hydrogenation catalyst). Then, the amidated tertiary amine (XXV) is esterified with a fatty acid (XXVI) into an ester of amidated tertiary amine (XXVII). The fatty acid (XXVI) to be used in this esterification is generally one resulting from a natural fat or oil such

as coconut oil, palm oil, tallow, rape seed oil, fish oil or the like, or alternatively may be a synthetic fatty acid having 8 to 24 carbon atoms.

The ester of amidated tertiary amine (XXVII) thus prepared is quaternized with an quaternizing agent (XXVIII) in a conventional manner, followed by, if necessary, the replacement of the counter ion. Thus, a quaternary ammonium salt (X) is obtained. The quaternizing agent (XXVIII) usable in this case includes alkyl halides (such as methyl chloride, ethyl chloride and methyl bromide) and dialkylsulfuric acids (such as dimethylsulfuric acid and diethylsulfuric acid). The quaternization may be conducted in a solvent and examples of the solvent include lower alcohols such as ethanol, isopropanol and butanol, the softener base material (1) of the present invention, the component (A) of the softener base material (2) of the present invention and mixtures of two or more of them. The quaternary ammonium salt (X) thus prepared may be, if necessary, subjected to the replacement of the counter ion with an ion exchange resin or the like.

Examples of the component (B-1) are as follows:

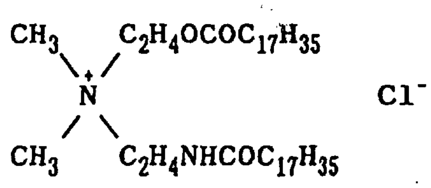
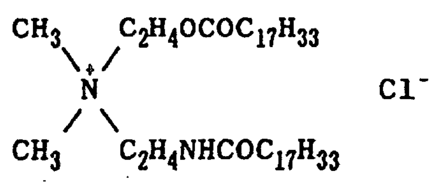

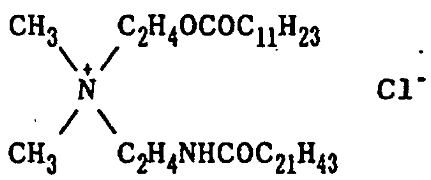
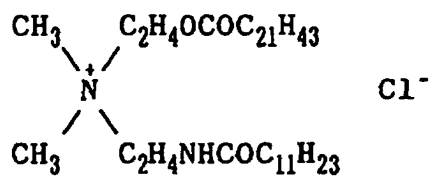

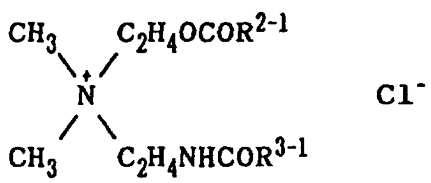
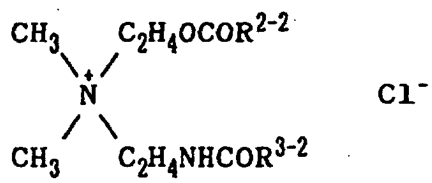

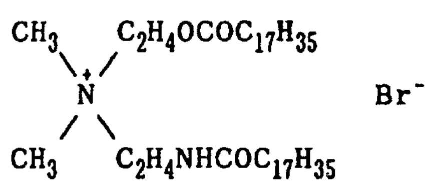
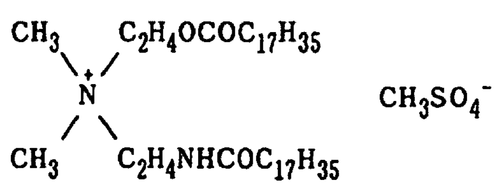

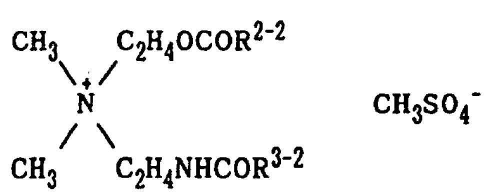

[wherein

$R^{2-1}$ and $R^{3-1}$: each an alkyl group resulting from hardened tallow fatty acid, and
$R^{2-2}$ and $R^{3-2}$: each an alkyl group resulting from hardened palm oil fatty acid]

<Component (B-2)>

The component (B-2) according to the present invention is a compound represented by the general formula (XI).
In the general formula (XI) $Y^-$ is a halide ion (such as $Cl^-$, $Br^-$ or the like), $CH_3SO_4^-$, $C_2H_5SO_4^-$, $C_3H_7SO_4^-$ or the like.
Examples of the component (B-2) are as follows:

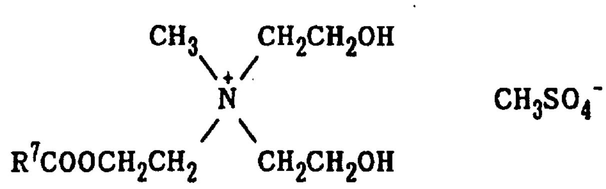

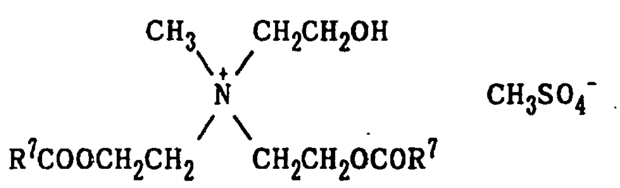

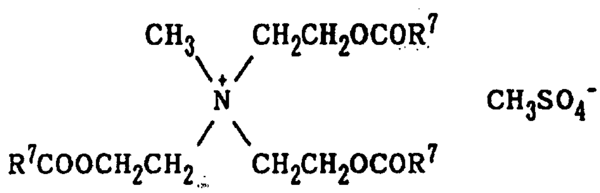

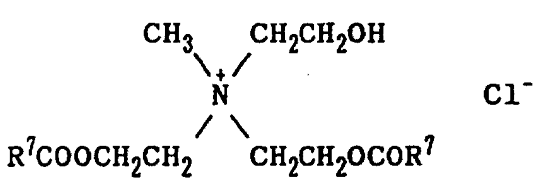

[wherein $R^7$: as defined above].
The component (B-2) can be prepared by, e.g., esterifying triethanolamine with a fatty acid and quaternizing the obtained ester. The solvent usable in this quaternization includes lower alcohols such as ethanol, isopropanol and butanol, the softener base material (1) of the present invention, the component (A) of the softener base material (2) of the present invention and mixture of two or more of them.

[Softener composition (1)]

The softener composition of the present invention comprises the above softener base material (1) or (2) and water.
Although the softener base material (1) can be used alone as a softener base material, the softener base

material (1) may be used together with the component (B) of the softener base material (2). When the softener base material (1) is used together with the component (B) of the softener base material (2), it is desirable that the weight ratio of the softener base material (1) to the component (B) of the softener base material (2) ranges from 2/1 to 1/9, more desirably 1/1 to 1/9, most desirably 1/2 to 1/9. When the weight ratio lies within this range, a particularly excellent softening effect can be attained.

According to the present invention, the softener base material (1) or (2) or the softener base materials (1) and (2) (hereinafter referred to as "the softener base material (1) and/or (2) is used in an amount of 3 to 40% by weight, preferably 5 to 30% by weight, still preferably 5 to 25% by weight based on the softener composition.

When the amount of the softener base material (1) and/or (2) lies within this range, a particularly desirable softening effect according to the present invention can be attained, and

the resulting softener composition will not have too high a viscosity to be easily taken out of a bottle unfavorably.

Among the softener compositions according to the present invention, one containing the softener base material (1) and/or (2) in a larger amount tends to thicken during storage. In order to prevent such thickening, the softener composition of the present invention may further contain, as component (C), a polyether compound prepared by the addition reaction of a compound having at least three active hydrogen atoms with an alkylene oxide having 2 or 3 carbon atoms and having an average molecular weight of 5,000 to 2,000,000 (wherein the total amount of ethylene oxide moieties is 55% by weight or above based on the component (C)), or a derivative thereof.

Examples of the compound having at least three active hydrogen atoms to be used as the starting material for preparing the component (C) include polyhydric alcohols such as trimethylolpropane, diethanolamine, triethanolamine, glycerol, pentaerythritol, sorbitol, sucrose, polyglycerol, polyvinyl alcohol and partially saponified polyvinyl acetate; polyhydric phenols such as phenolic resins and alkylphenol-formalin condensates; and polyamine compounds such as polyethyleneimines (e.g., ethylenediamine, diethylenetriamine, triethylenetetramine, tetraethylenepentamine and pentaethylenehexamine). Further, partially amidated derivatives and N-alkylated derivatives of these polyamine compounds can be used, as far as they have at least three active hydrogen atoms.

The component (C) can be prepared by conducting the addition reaction of a compound having at least three active hydrogen atoms with an alkylene oxide component containing ethylene oxide as an essential component in a conventional manner. In particular, the component (C) is preferably an adduct of the compound with ethylene oxide alone or an adduct thereof with both ethylene oxide and propylene oxide wherein the whole or part of the ethylene oxide and propylene.oxide molecules are arranged in block. Although the order of the addition reactions may be arbitrary, an adduct prepared by conducting the addition reaction of a compound having at least three active hydrogen atoms with propylene oxide (hereinafter abbreviated to "PO") and thereafter the addition reaction of the obtained adduct with ethylene oxide (hereinafter abbreviated to "EO") is preferable, because a softener composition comprising such an adduct is inhibited from thickening during storage even when the content of the base material of the present invention is high.

The molecular weight of the component (C) may range from 5,000 to 2,000,000, preferably 10,000 to 100,000. Further, the total weight of oxyethylene moieties (EO chains) accounts for at least 55% by weight, preferably at least 80% by weight of the molecular weight of the component (C).

When the molecular weight of the component (C) is less than 5,000, the resulting softener composition will not be sufficiently inhibited from thickening during storage, while when it exceeds 2,000,000, the resulting softener composition will have too high a viscosity to be easily taken out of a bottle unfavorably.

When the total weight of the oxyethylene moieties accounts for less than 55% by weight of the molecular weight of the component (C), the resulting softener composition will not sufficiently be inhibited from thickening during storage.

The derivative of the polyether compound usable as the component (C) includes products of crosslinking of the polyether compound with isocyanate compounds; products prepared by sulfonating, phosphorylating and carboxyalkylating the polyether compound at the terminal hydroxy group; products prepared by esterifying the terminal hydroxy group of the polyether compound with fatty acids; and cationic products prepared by converting a part of the nitrogen atoms of the polyether compound into cations. Among these derivatives, products prepared by esterifying the terminal hydroxy group of the polyether compound with fatty acids and cationic products prepared by converting a part of the nitrogen atoms of the polyether compound into cations are particularly preferable.

The fatty acid to be used in the above esterification is preferably one having 7 to 23 carbon atoms. The number of double bonds of the fatty acid or whether the fatty acid has a branch or not has not any great influence on the performance.

The cationic products include products prepared by converting the polyether compound into cations with dialkylsulfuric acids and alkyl halides and those prepared by neutralizing it with acetic acid and alkylbenzenesulfonic acid.

In order to attain both the desirable softening performance according to the present invention and the inhibition of the softener composition from thickening during storage simultaneously, it is desirable that the amount of the component (C) is so adjusted that the amount of the component (C) is 0.5 to 5% by weight, preferably 1 to 3% by weight based on the softener composition, that the weight ratio of the component (C) to the softener base material (1) and/or (2) is 1/100 to 1/2.5, preferably 1/50 to 1/5, and that the total amount of the softener base material of the present invention and the component (C) is 3.5 to 45% by weight or 4 to 50% by weight, preferably 11 to 40% by weight, still preferably 14 to 35% by weight based on the softener composition.

The softener composition of the present invention may further contain a linear or branched, saturated or unsaturated fatty acid having 8 to 24 carbon atoms as component (D) to improve the softening performance and storage stability of the composition. It is preferable that the weight ratio of the component (D) to the softener base material (1) and/or (2) range from 0.5/100 to 1/1, still preferably 1/100 to 1/2. Further, it is preferable that the total amount of the softener base material (1) and/or (2) and the component (D) be 4 to 50% by weight based on the softerner composition. Examples of the fatty acid to be used here include caprinic acid, lauric acid, myristic acid, palmitic acid, oleic acid, stearic acid, isostearic acid, arachidic acid, behenic acid, fatty acids prepared from coconut oil, palm kernel oil, unhardened and hardened tallows, lard, palm oil, rape seed oil, fish oil, palm stearin and hardened palm stearin, and mixtures of two or more of them.

Further, the softener composition of the present invention may contain a softener base material represented by the following general formula (XXX-1), (XXX-2) or (XXX-3) and the amount of this softerner base material is 0 to 15% by weight based on the composition:

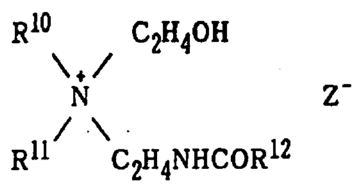

(XXX-1)

[wherein

$R^{10}$ and $R^{11}$: each an alkyl group having 1 to 4 carbon atoms,
$R^{12}$: a linear or branched alkyl or alkenyl group having 7 to 23 carbon atoms, and
$Z^-$: a halide ion or an $R^8SO_4^-$ group wherein $R^8$ represents an alkyl group having 1 to 4 carbon atoms and $Z^-$ and $X^-$ may be the same or different from each other],

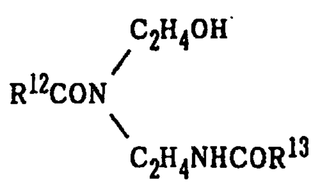

(XXX-2)

[wherein

$R^{12}$: as defined above, and
$R^{13}$: a linear or branched alkyl or alkenyl group having 7 to 23 carbon atoms], and

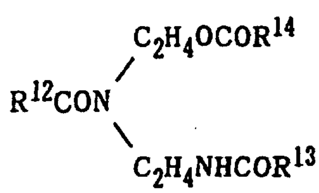

(XXX-3)

[wherein

$R^{12}$ and $R^{13}$: each as defined above, and
$R^{14}$: a linear or branched alkyl or alkenyl group having 7 to 23 carbon atoms].

Further, the softener composition of the present invention may further contain a conventional softener, so far as the object of the present invention is not marred.

The softener composition of the present invention may further contain an electrolyte such as NaCl, $CaCl_2$, $MgCl_2$ or N-methyl-N,N,N-triethanolammonium salt to control the viscosity of the composition. The amount of the electrolyte used is 0 to 5% by weight, preferably 0.01 to 2% by weight based on the composition.

The softener composition of the present invention may further contain an acid or an alkali to regulate the pH of the composition. It is desirable from the standpoints of the viscosity and storage stability of the composition that the pH of the composition be regulated within the range of 1.5 to 6.5.

Although the softener composition of the present invention is highly stable to long-term storage, the softener composition may further contain a nonionic surfactant such as polyoxyethylene(5-50 mole) alkyl or alkenyl($C_{12}$-$C_{24}$) ether or polyoxyethylene(5-50 mole) alkyl or alkenyl($C_{12}$-$C_{24}$)amine; a hydrotrop or hydrotropic agent or dispersing agent such as ethylene glycol, propylene glycol, glycerol or urea; or a lower alcohol such as ethanol or isopropyl alcohol for the purpose of enhancing the stability of the composition under severer storage conditions. Further, the composition of the present invention may further contain a pigment or a dye for the purpose of improving the appearance of the composition, a silicone for the purpose of improving the antifoaming properties in rinsing, and/or a perfume for the purpose of improving the comfortableness in using or that of the treated clothes.

The softener composition of the present invention comprises the above essential and optional components and the balance of water.

A process for preparing the softener composition of the present invention will now be described, though the present invention is not limited to this process. Namely, a mixture comprising the softener base material of the present invention and optional components (exclusive of the component (C)) is molten and gradually dropped into ion-exchanged water kept at 60°C by heating under stirring to form an emulsion, followed by if necessary, the addition of the component (C). A nonionic surfactant may be preliminarily added to the ion-exchanged water and an inorganic salt may be added after the addition of the softener base material of the present invention and optional components to control the viscosity of the composition.

<u>Example</u>

The invention will now be described by referring to the following Examples.

In the Examples, all percentages are by weight unless otherwise noted, and the balance is composed of water.

Examples 1 to 41

Liquid softener compositions having formulations specified in Tables 9 to 12 were prepared by the use of softener base materials (1) listed in Tables 1 and 2, components (A) of the softener base material (2) listed in Table 3, components (B-1) of the softener base material (2) listed in Table 4, components (B-2) of the softener base material (2) listed in Table 5, components (C) listed in Table 6, components (D) listed in Table 7 and other components listed in Table 8.

Comparative Example 1

A liquid softener composition having a formulation specified in Table 13 was prepared by the use of a mixture comprising the softener base material (1-6) listed in Table 1 and the component (E-5) listed in Table 8 (corresponding to the mixture of Example 12 of Japanese Patent Laid-Open No. 57632/1994).

Comparative Example 2

A liquid softener composition having a formulation specified in Table 13 was prepared by the use of a mixture comprising the softener base material (B-2-3) listed in Table 5 and the component (E-6) listed in Table 8 (corresponding to the mixture of Example 4 of Japanese Patent Laid-Open No. 10263/1994).

Comparative Examples 3 to 16

Liquid softener compositions having formulations specified in Tables 13 and 14 were prepared by the use of components (A) of the softener base material (2) listed in Table 3, components (B-1) of the·softener base material (2) listed in Table 4 and other components listed in Table 8.

The pieces of cloth treated with the liquid softener compositions prepared in Examples 1 to 41 and Comparative Examples 1 to 16 were evaluated for softness and greasiness by the following method. Further, the compositions

were also evaluated for storage stablility by the following method. The results are given in Tables 9 to 14.

<Evaluation of softness and greasiness>

Commercially available cotton towel, acrylic fabric and polyester fabric were washed with "Attack" (a product of Kao Corporation, registered trademark) five times and thereafter freed from the detergent. The resulting towel and fabrics were treated with 0.5% (by weight based on the towel or fabric) of each of the liquid softener compositions prepared above at 25°C and a bath ratio of 1/10 under stirring for 3 minutes. The towel and fabrics thus treated were air-dried in room and allowed to stand in a thermo-hygrostatic chamber of 20°C and 65% RH for 24 hours.
The resulting towel and fabrics were evaluated for softness and gresiness.
The evaluation of softness and greasiness was conducted according to the following criteria as compared with the towel and fabrics treated with the softener composition of Comparative Example 16 (as control)

softness:

+2: softer than the control
+1: somewhat softer than the control
0: equivalent to the control
- 1: the control is somewhat softer
-2: the control is softer

greasiness:

+2: the control is greasier
+1: the control is somewhat greasier
0: equivalent to the control
-1: somewhat greasier than the control
- 2: greasier than the control

<Evaluation of storage stability>

The liquid softener compositions prepared above were stored in a hermetically stored state at 20°C for 20 days and evaluated for appearance and fluidity with the naked eye under hermetically sealed conditions. A case wherein no change in the appearance or the fluidity was shown by "good", while a case wherein a change was observed was shown by the change.

<Evaluation of water absorption properties [Byreck method]>

The pieces of cloth treated in the same manner as that described in the section "Evaluation of softness and greasiness" were allowed to stand in a thermo-hygrostatic chamber of 25°C and 65% RH for 24 hours. The resulting cotton towel was evaluated for water absorption properties.
A rectangular piece (25 cm × 2 cm) was cut off from the non-pile area of the cotton towel. Water of 25°C was placed in the above thermo-hygrostatic chamber and this piece was dipped in the water in a state kept vertically up to 2 cm from the lower end to absorb water. After 15 minutes, the height of ascent of water was measured.

Table 1

| | Softener base material (1) | | |
|---|---|---|---|
| | Synth. process | (I)/(II)/(III)/(IV) wt. ratio | Griffin's HLB |
| 1-1 | compound prepared from methyl ester of hardened palm stearic acid, glycerol and EO*1 at molar ratio of 2 : 1 : 4 by process (ii) | 0.150/0.444/0.393/0.013 | 6.69 |
| 1-2 | compound prepared from semihardened tallow*2. glycerol and EO at a molar ratio of 1 : 0.2 : 9.6 by process (iv) | 0.045/0. 306/0.647/0.002 | 7.80 |
| 1-3 | compound prepared from tallow, glycerol and EO at a molar ratio of 1 : 1 : 8 by process (ii) | 0.301/0.449/0.199/0.051 | 7.82 |

Table 1 (continued)

| | Softener base material (1) | | |
|---|---|---|---|
| | Synth. process | (I)/(II)/(III)/(IV) wt. ratio | Griffin's HLB |
| 1-4 | compound prepared from palm stearin, glycerol and EO at a molar ratio of 1 : 0.5 : 12 by process (ii) | 0.164/0.444/0.375/0.017 | 9.09 |
| 1-5 | compound prepared from tallow, glycerol and EO at a molar ratio of 2.5 : 1 : 12 by process (iii) | 0.048/0.312/0.638/0.002 | 9.43 |
| 1-6 | compound prepared from hardened tallow, glycerol and EO at a molar ratio of 1 : 0.25 : 15 by process (ii) [the component (b-1) of Example of Japanese Patent Laid-Open No. 57632/1994] | 0.068/0.352/0.576/0.004 | 9.63 |
| 1-7 | compound prepared from methyl ester of palm kernel oil fatty acid, glycerol and EO at a molar ratio of 1.7 : 1 : 6 by process (iv) | 0.255/0.454/0.250/0.041 | 9.76 |

Table 2

| | Softener base material (1) | | |
|---|---|---|---|
| | Synth. process | (I)/(II)/(III)/(IV) wt. ratio | Griffin's HLB |
| 1-8 | compound prepared from tallow fatty acid, glycerol and EO at a molar ratio of 2 : 1 : 10 by process (i) | 0.167/0.444/0.370/0.019 | 9.78 |
| 1-9 | compound prepared from methyl ester*3 of semihardened palm stearic acid, glycerol and EO at a molar ratio of 1.8 : 1 : 10 by process (iv) | 0.230/0.454/0.282/0.035 | 10. 52 |
| 1-10 | compound prepared from semihardened tallow. glycerol and EO at a molar ratio of 1 : 0.5 : 18 by process (ii) | 0.172/0.444/0.364/0.020 | 10.54 |
| 1-11 | compound prepared from methyl ester of palm kernel oil fatty acid, glycerol and EO at a molar ratio of 2 : 1 : 10 by process (ii) | 0.174/0.444/0.360/0.021 | 10.95 |
| 1-12 | compound prepared from hardened palm stearin, glycerol and EO at a molar ratio of 1 : 0.25 : 20 by process (ii) | 0.072/0.357/0.567/0.005 | 11.09 |
| 1-13 | compound prepared from coconut oil fatty acid, glycerol and EO at a molar ratio of 2 : 1 : 10 by process (iii) | 0.176/0.444/0.358/0.022 | 11.18 |
| 1-14 | compound prepared from palm oil fatty acid, glycerol and EO at a molar ratio of 2 : 1 : 16 by process (iii) | 0.178/0.444/0.355/0.022 | 11.79 |
| 1-15 | compound prepared from palm oil. glycerol and EO at a molar ratio of 1 : 1 : 24 by process (ii) | 0.327/0.423/0.173/0.077 | 11.97 |

notes)
*1: EO represents ethylene oxide
*2: semihardened tallow is a mixture comprising tallow and hardened tallow at a weight ratio of 1 : 1
*3: methyl ester of semihardened palm stearic acid is a mixture comprising methyl ester of palm stearic acid and methyl ester of hardened palm stearic acid at a weight ratio of 1 : 1

Table 3

| | Component (A) of the softener base material (2) | | |
|---|---|---|---|
| | Synth. process | (V)/(VI)/(VII)/(VIII) wt. ratio | Griffin's HLB |
| A-1 | compound prepared from methyl ester of tallow fatty acid, glycerol and EO at a molar ratio of 0.6 : 1 : 3 by process (iv) | 0.489/0.188/0.021/0.302 | 11.46 |
| A-2 | compound prepared from palm stearin, glycerol and EO at a molar ratio of 1 : 4.45 : 16.36 by process (i) | 0.460/0.165/0.017/0.338 | 12.09 |

Table 3 (continued)

| | Component (A) of the softener base material (2) | | |
|---|---|---|---|
| | Synth. process | (V)/(VI)/(VII)/(VIII) wt. ratio | Griffin's HLB |
| A-3 | compound prepared from hardened tallow fatty acid, glycerol and EO at a molar ratio of 2 : 1 : 20 by process (i) | 0.183/0.444/0.348/0.024 | 12.72 |
| A-4 | compound prepared from hardened palm stearin, glycerol and EO at a molar ratio of 1 : 0.36 : 40.91 by process (iii) | 0.133/0.419/0.434/0.014 | 14.14 |
| A-5 | compound prepared from ethyl laurate, glycerol and EO at a molar ratio of 0.55 : 1 : 4 by process (ii) | 0.448/0.141/0.014/0.397 | 14.18 |
| A-6 | compound prepared from coconut oil, glycerol and EO at a molar ratio of 1 : 2 : 36 by process (i) | 0.444/0.275/0.055/0.226 | 14.94 |

Table 4

| Component (B-1) of the softener base material (2) | |
|---|---|
| B-1-1[*1] | 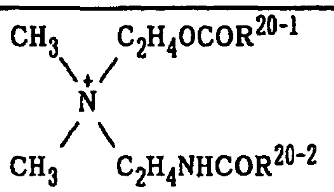 $Cl^-$ |
| B-1-2[*2] | 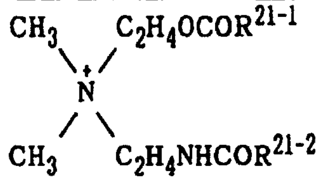 $Cl^-$ |
| B-1-3 | 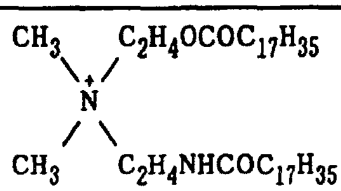 $Cl^-$ |
| B-1-4 | 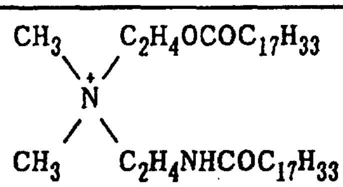 $Cl^-$ |

notes)

*1: $R^{20-1}$ and $R^{20-2}$ each represent an alkyl group resulting from hardened tallow fatty acid

*2: $R^{21-1}$ and $R^{21-2}$ each represent an alkyl group resulting from hardened palm oil fatty acid

Table 5

| Component (B-2) of the softener base material (2) | |
|---|---|
| B-2-1[*1] | 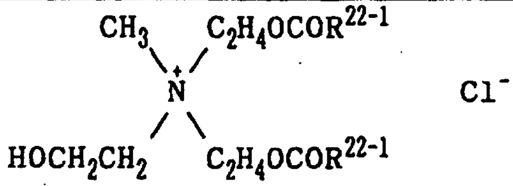 |
| B-2-2[*1] | mixture of the following components (weight ratio of monoester to diester to triester: 20 : 55 : 25) 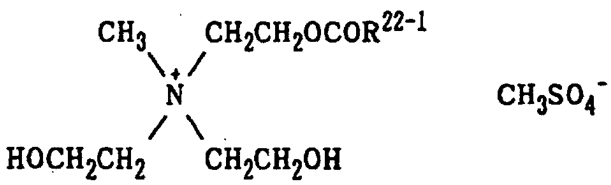 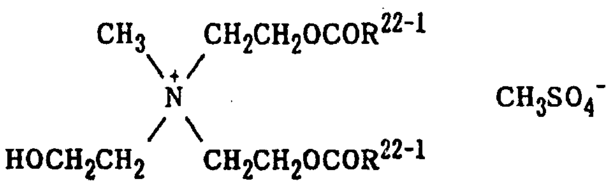 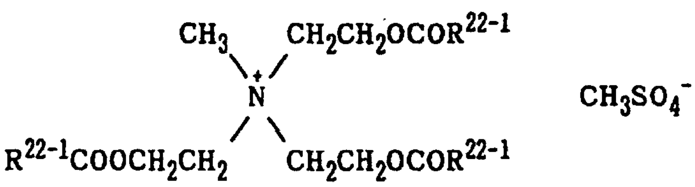 |

Table 5 (cont'd)

| Component (B-2) of the softener base material (2) | |
|---|---|
| B-2-3*2 | mixture of the following components (weight ratio of monoester to diester to triester: 20 : 55 : 25)<br>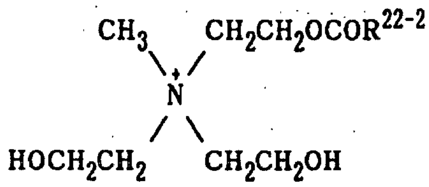 $CH_3SO_4^-$<br>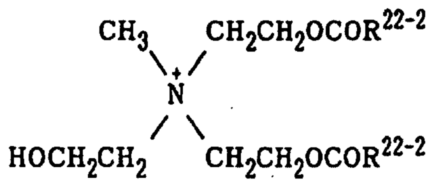 $CH_3SO_4^-$<br>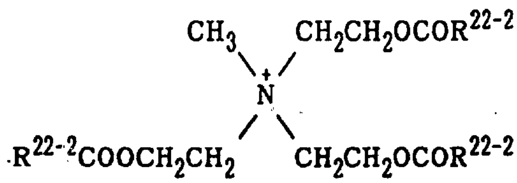 $CH_3SO_4^-$ |

notes)

*1: $R^{22-1}$ represents an alkyl group resulting from hardened tallow fatty acid

*2: $R^{22-2}$ represents an alkyl group resulting from a mixture comprising unhardened tallow fatty acid and hardened tallow fatty acid at a weight ratio of 75 : 25

Table 6

| Component (C) | |
|---|---|
| C-1 | adduct of glycerol with EO (MW 8,900) |
| C-2 | adduct of glycerol with PO/EO (15 : 85) (MW 10,000) |
| C-3 | adduct of sorbitol with PO/EO (10 : 90) (MW 15,000) |
| C-4 | adduct of tetraethylenepentamine with PO/EO (2 : 98) (MW 20,000) |
| C-5 | adduct of polyethyleneimine (MW: 3000) with PO/EO (5 : 95) (MW 300,000) |

Table 7

| Component (D) | |
|---|---|
| D- 1 | hardened tallow fatty acid |
| D- 2 | semihardened tallow fatty acid (wt. ratio of tallow to hardened tallow: 75 : 25) |
| D- 3 | palm oil fatty acid |
| D- 4 | palm stearic acid |
| D- 5 | stearic acid |
| D- 6 | oleic acid |
| D- 7 | hardened palm oil fatty acid |
| D- 8 | palm kernel oil fatty acid |
| D- 9 | coconut oil fatty acid |
| D-10 | lauric acid |
| D-11 | tallow fatty acid |
| D-12 | hardened palm stearic acid |

Table 8

| Other components | |
|---|---|
| E-1 | ethanol |
| E-2 | isopropanol |
| E-3 | polyoxyethylene (20 mole) layryl ether |
| E-4 | polyoxyethylene(20 mole)stearylamine |
| E-5 | 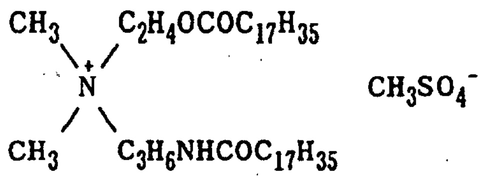 [the component (a-3) of Example of Japanese Patent Laid-Open No. 57632/1994] |
| E-6 | product of the reaction of lard oil with 15 wt% of propylene oxide [the component described in Example 4 of Japanese Patent Laid-Open No. 10263/1994] |
| E-7*1 | compound prepared by reacting tallow fatty acid with glycerol and EO at a molar ratio of 1.8 : 1 : 50 by process (i) (I)/(II)/(III)/(IV) w ratio : 0.266/0.440/0.240/0.053 HLB=16.42 |
| E-8 | compound prepared by reacting hardened palm oil with glycerol and EO at a molar ratio of 1 : 0.111 : 20 by process (ii) (I)/(II)/(III)/(IV) wt. ratio: 0.019/0.215/0.765/0.001 HLB=10.83 |
| E-9 | compound prepared by reacting palm kernel oil with glycerol and EO at a molar ratio of 1 : 0.111 : 6.67 by process (ii) (I)/(II)/(III)/(IV) wt. ratio: 0.017/0.205/0.778/0.000 HLB=7.5 |

note)

*1: EO represents ethylene oxide

Table 9

| | | Softener base material (1) | Softener base material (2) | | Component (C) | Component (D) | Other component | Softness | Greasiness | Storage stability | Water absorption properties (cm) |
|---|---|---|---|---|---|---|---|---|---|---|---|
| | | | Component (A) | Component (B) | | | | | | | |
| Ex. | 1 | 1-9 (20) | - | - | - | - | - | 0 | +2 | good | 15.5 |
| | 2 | 1-10 (20) | - | - | - | - | - | 0 | +2 | good | 15.5 |
| | 3 | 1-1 (20) | - | - | - | - | E-3 (3) | 0 | +2 | good | 13.2 |
| | 4 | 1-2 (20) | - | - | - | - | E-3 (3) | 0 | +2 | good | 14.0 |
| | 5 | 1-3 (20) | - | - | - | - | E-3 (3) | 0 | +2 | good | 14.5 |
| | 6 | 1-4 (20) | - | - | - | - | E-3 (2) | 0 | +2 | good | 14.5 |
| | 7 | 1-5 (20) | - | - | - | - | E-3 (2) | 0 | +2 | good | 15.0 |
| | 8 | 1-6 (20) | - | - | - | - | E-3 (2) | 0 | +2 | good | 14.5 |
| | 9 | 1-7 (20) | - | - | - | - | E-3 (2) | 0 | +2 | good | 15.2 |
| | 10 | 1-8 (20) | - | - | - | - | E-3 (2) | 0 | +2 | good | 15.2 |
| | 11 | 1-9 (18) | - | - | - | D-3 (2) | E-3 (2) | 0 | +2 | good | 15.0 |

Table 10

| | | Softener base material (1) | Softener base material (2) | | Component (C) | Component (D) | Other component | Softness | Greasiness | Storage stabilty | Water absorption properties (cm) |
|---|---|---|---|---|---|---|---|---|---|---|---|
| | | | Component (A) | Component (B) | | | | | | | |
| Ex. | 12 | 1-10 (18) | - | - | - | D-9 (2) | E-3 (2) | 0 | +2 | good | 15.0 |
| | 13 | 1-11 (19.5) | - | - | - | D-8 (0.5) | - | 0 | +2 | good | 15.7 |
| | 14 | 1-12 (19.5) | - | - | - | D-12 (0.5) | - | 0 | +2 | good | 15.2 |
| | 15 | 1-13 (19.5) | - | - | - | D-10 (0.5) | - | 0 | +2 | good | 15.9 |
| | 16 | 1-14 (19) | - | - | - | D-1 (1) | - | 0 | +2 | good | 15.9 |
| | 17 | 1-15 (18.5) | - | - | - | D-4 (1.5) | - | 0 | +2 | good | 16.0 |
| | 18 | - | A-1 (4) | B-1-1 (16) | - | - | E-2 (2) | +2 | +2 | good | 13.0 |
| | 19 | - | A-2 (4) | B-1-1 (16) | - | - | E-2 (2) | +2 | +2 | good | 13.5 |
| | 20 | - | A-3 (4) | B-1-1 (16) | - | - | E-2 (2) | +2 | +2 | good | 13.2 |
| | 21 | - | A-4 (4) | B-1-1 (16) | - | - | E-2 (2) | +2 | +2 | good | 13.2 |
| | 22 | - | A-5 (4) | B-1-1 (16) | - | - | E-2 (2) | +2 | +2 | good | 13.2 |

Table 11

| | | Softener base material (1) | Softener base material (2) | | Component (C) | Component (D) | Other component | Softness | Greasiness | Storage stabilty | Water absorption properties (cm) |
|---|---|---|---|---|---|---|---|---|---|---|---|
| | | | Component (A) | Component (B) | | | | | | | |
| Ex. | 23 | - | A-6 (4) | B-1-1 (16) | - | - | E-2 (2) | +2 | +2 | good | 13.7 |
| | 24 | 1-15 (4) | - | B-1-1 (16) | - | - | E-2 (2) | +2 | +2 | good | 13.5 |
| | 25 | 1-15 (4) | - | B-1-2 (16) | - | - | E-2 (2) | +2 | +2 | good | 13.5 |
| | 26 | 1-15 (4) | - | B-1-3 (16) | - | - | E-2 (2) | +2 | +2 | good | 13.6 |
| | 27 | 1-15 (4) | - | B-1-4 (16) | - | - | E-2 (2) | +2 | +2 | good | 14.5 |
| | 28 | - | A-1 (4) | B-2-3 (16) | - | - | E-2 (2) | +2 | +2 | good | 13.2 |
| | 29 | - | A-2 (4) | B-2-3 (16) | - | - | E-2 (2) | +2 | +2 | good | 13.7 |
| | 30 | - | A-3 (4) | B-2-3 (16) | - | - | E-2 (2) | +2 | +2 | good | 13.5 |
| | 31 | - | A-4 (4) | B-2-3 (16) | - | - | E-2 (2) | +2 | +2 | good | 13.5 |
| | 32 | - | A-5 (4) | B-2-3 (16) | - | - | E-2 (2) | +2 | +2 | good | 13.5 |
| | 33 | - | A-6 (4) | B-2-3 (16) | - | - | E-2 (2) | +2 | +2 | good | 14.0 |

Table 12

| | | Softener base material (1) | Softener base material (2) | | Component (C) | Component (D) | Other component | Softness | Greasiness | Storage stabilty | Water absorption properties (cm) |
|---|---|---|---|---|---|---|---|---|---|---|---|
| | | | Component (A) | Component (B) | | | | | | | |
| Ex. | 34 | 1-15 (4) | - | B-2-1 (16) | - | - | E-2 (2) | +2 | +2 | good | 13.2 |
| | 35 | 1-15 (4) | - | B-2-2 (16) | - | - | E-2 (2) | +2 | +2 | good | 13.2 |
| | 36 | 1-15 (4) | - | B-2-3 (16) | - | - | E-2 (2) | +2 | +2 | good | 13.5 |
| | 37 | 1-5 (2) | - | B-1-1 (18) | C-1 (1.5) | D-2 (4) | E-2 (2) E-3 (2) | +2 | +2 | good | 13.2 |
| | 38 | 1-5 (2) | - | B-1-1 (18) | C-2 (1.5) | D-5 (4) | E-2 (2) E-3 (2) | +2 | +2 | good | 13.0 |
| | 39 | 1-5 (2) | - | B-1-1 (18) | C-3 (1.5) | D-6 (4) | E-2 (2) E-3 (2) | +2 | +2 | good | 13.2 |
| | 40 | 1-10 (3) | - | B-2-3 (17) | C-4 (1.5) | D-7 (4) | E-2 (2) E-3 (1) E-4 (1) | +2 | +2 | good | 13.0 |
| | 41 | 1-10 (3) | - | B-2-3 (17) | C-5 (1.5) | D-11 (4) | E-2 (2) E-3 (1) E-4 (1) | +2 | +2 | good | 13.2 |

Table 13

| | | Softener base material (1) | Softener base material (2) | | Component (C) | Component (D) | Other component | Softness | Greasiness | Storage stabilty | Water absorption properties (cm) |
|---|---|---|---|---|---|---|---|---|---|---|---|
| | | | Component (A) | Component (B) | | | | | | | |
| Comp. Ex. | 1 | - | A-1 (2) | - | - | - | E-5 (18) | -1 | -1 | good | 11.0 |
| | 2 | - | - | B-2-3 (16) | - | - | E-6 (4) | -1 | +1 | separati-on | 11.5 |
| | 3 | - | A-1 (20) | - | - | - | - | -1 | +2 | separati-on | 15.9 |
| | 4 | - | A-2 (20) | - | - | - | - | -1 | +2 | separati-on | 16.0 |
| | 5 | - | A-3 (20) | - | - | - | - | -2 | +2 | separati-on | 16.0 |
| | 6 | - | A-4 (20) | - | - | - | - | -2 | +2 | separati-on | 16.0 |
| | 7 | - | A-5 (20) | - | - | - | - | -2 | +2 | separati-on | 16.0 |
| | 8 | - | A-6 (20) | - | - | - | - | -2 | +2 | separati-on | 16.0 |
| | 9 | - | - | B-1-1 (16) | - | - | E-1 (2) E-3 (2) E-7 (4) | -1 | +2 | good | 13.7 |
| | 10 | - | - | B-2-3 (16) | - | - | E-1 (2) E-3 (2) E-7 (4) | -1 | +2 | good | 14.0 |

Table 14

| | | Softener base material (1) | Softener base material (2) | | Component (C) | Component (D) | Other component | Softness | Greasiness | Storage stability | Water absorption properties (cm) |
|---|---|---|---|---|---|---|---|---|---|---|---|
| | | | Component (A) | Component (B) | | | | | | | |
| Comp. Ex. | 11 | - | - | B-1-1 (16) | - | - | E-2 (2) E-3 (2) E-8 (4) | 0 | +2 | separation | 12.0 |
| | 12 | - | - | B-2-3 (16) | - | - | E-2 (2) E-3 (2) E-8 (4) | 0 | +2 | separation | 13.2 |
| | 13 | - | - | B-1-1 (16) | - | - | E-2 (2) E-3 (2) E-9 (4) | 0 | +2 | separation | 11.0 |
| | 14 | - | - | B-2-3 (16) | - | - | E-2 (2) E-3 (2) E-9 (4) | 0 | +2 | separation | 11.2 |
| | 15 | - | - | B-1-1 (20) | - | - | E-2 (2) | +2 | -2 | good | 10.5 |
| | 16 | - | - | B-2-3 (20) | - | - | E-2 (2) | 0 | 0 | gelation | 10.8 |

note) In Tables 9 to 14, the figures in parentheses each represent the content of a corresponding component in the composition and the unit thereof is % by weight. The balance is composed of water.

As apparent from the above results, the softener compositions (1) and (2) of the present invention can impart excellent softness and water absorption properties to cloth without making the cloth greasy to the touch, and are excellent in storage stability.

## Claims

1. A liquid softener composition **characterized by** comprising the following softener base material (1) or (2) and water:

[softener base material (1)]

a mixture comprising a compound represented by the following general formula (I), a compound represented by the following general formula (II), a compound represented by the following general formula (III) and a compound represented by the following general formula (IV), provided that

the weight ratio of the compound (I) to the total sum of the compounds (I), (II), (III) and (IV) ranges from 0.040 to 0.327,
the weight ratio of the compound (II) to the total sum of the compounds (I), (II), (III) and (IV) ranges from 0.298 to 0.469,
the weight ratio of the compound (III) to the total sum of the compounds (I), (II), (III) and (IV) ranges from 0.173 to 0.661, and
the weight ratio of the compound (IV) to the total sum of the compounds (I), (II), (III) and (IV) ranges from 0.001 to 0.077,

and that the mixture comprising the compounds (I), (II), (III) and (IV) has a Griffin's HLB value ranging from 5 to 12:

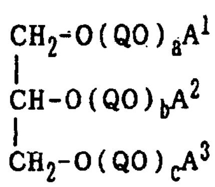

(I)

[wherein

A¹, A² and A³: each an RCO group or a hydrogen atom with the proviso that one of $A^1$, $A^2$ and $A^3$ is an RCO group and the others thereof are each a hydrogen atom, wherein R represents a linear or branched alkyl or alkenyl group having 7 to 23 carbon atoms,
Q: an alkylene group having 2 or 3 carbon atoms or an alkylene group mixture comprising one having 2 carbon atoms and one having 3 carbon atoms, and
a, b and c: each a number of 0 or above, provided that the sum of a, b and c is 4 to 18 on the average].

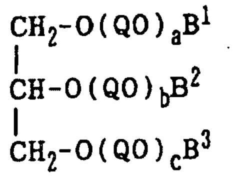

(II)

[wherein

$B^1$, $B^2$ and $B^3$: each an RCO group or a hydrogen atom with the proviso that two of $B^1$, $B^2$ and $B^3$ are each an RCO group and the other thereof is a hydrogen atom, wherein R is as defined above, and
Q, a, b and c: each as defined above],

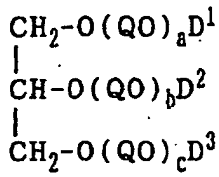

(III)

[wherein

$D^1$, $D^2$ and $D^3$: each an RCO group wherein R is as defined above, and
Q, a, b and c: each as defined above], and

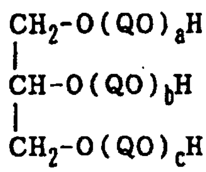

(IV)

[wherein

Q, a, b and c: each as defined above], and

[softener base material (2)]
a mixture comprising the following components (A) and (B) wherein the weight ratio of the component (A) to the component (B) ranges from 2/1 to 1/9:

<component (A)>
a mixture comprising a compound represented by the following general formula (V), a compound represented by the following general formula (VI), a compound represented by the following general formula (VII) and a compound represented by the following general formula (VIII),
provided that

the weight ratio of the compound (V) to the total sum of the compounds (V), (VI), (VII) and (VIII) ranges from 0.040 to 0.527,
the weight ratio of the compound (VI) to the total sum of the compounds (V), (VI), (VII) and (VIII) ranges from 0.133 to 0.469,
the weight ratio of the compound (VII) to the total sum of the compounds (V), (VI), (VII) and (VIII) ranges from 0.013 to 0.661, and
the weight ratio of the compound (VIII) to the total sum of the compounds (V), (VI), (VII) and (VIII) ranges from 0.001 to 0.417,

and that the mixture comprising the compounds (V), (VI), (VII) and (VIII) has a Griffin's HLB value ranging

from 5 to 15, except the mixtures falling under the category of the softener base material (1):

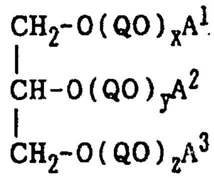

(V)

[wherein

$A^1$, $A^2$, $A^3$ and Q: each as defined above, and

x, y and z: each a number of 0 or above, provided that the sum of x, y and z is 1 to 50 on the average],

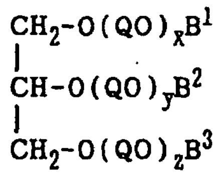

(VI)

[wherein

$B^1$, $B^2$, $B^3$, Q, x, y and z: each as defined above]

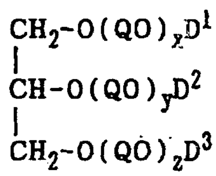

(VII)

[wherein

$D^1$, $D^2$, $D^3$, Q, x, y and z: each as defined above]

and

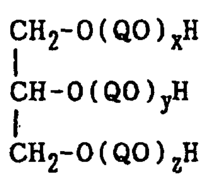

(VIII)

[wherein

Q, x, y and z: each as defined above], and

<component (B)>
at least one member selected from the following components (B-1) and (B-2):

<<component (B-1)>>
a compound represented by the following general formula (X):

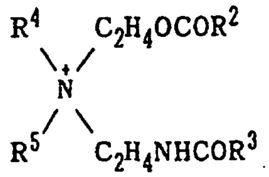 $X^-$ (X)

[wherein

$R^2$ and $R^3$: the same or different from each other a linear or branched alkyl or alkenyl group having 7 to 23 carbon atoms,
$R^4$ and $R^5$: each an alkyl group having 1 to 4 carbon atoms, and
$X^-$: a halogen ion or $R^4SO_4$ wherein $R^4$ is as defined above], and

<<component (B-2)>>
a compound represented by the following general formula (XI):

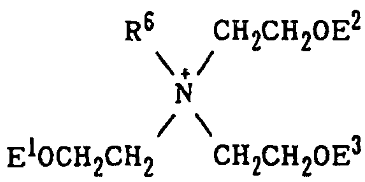 $Y^-$ (XI)

[wherein

$E^1$, $E^2$ and $E^3$: each an $R^7CO$ group or a hydrogen atom with the proviso that at least one of $E^1$, $E^2$ and $E^3$ is an $R^7CO$ group, wherein $R^7$ represents a linear or branched alkyl or alkenyl group having 7 to 23 carbon atoms,
$R^6$: an alkyl group having 1 to 4 carbon atoms, and
$Y^-$: a halogen ion or $R^6SO_4$ wherein $R^6$ is as defined above].

**2.** A liquid softener composition as set forth in claim 1, which contains a softener base material (1) comprising the compounds (I), (II), (III) and (IV) wherein

the weight ratio of the compound (I) to the sum total of the compounds (I), (II), (III) and (IV) ranges from 0.040 to 0.317,
the weight ratio of the compound (II) to the sum total of the compounds (I), (II), (III) and (IV) ranges from 0.298 to 0.469,
the weight ratio of the compound (III) to the sum total of the compounds (I), (II), (III) and (IV) ranges from 0.183 to 0.661,
the weight ratio of the compound (IV) to the sum total of the compounds (I), (II), (III) and (IV) ranges from 0.001 to 0.067,

and the sums of a, b and c in the general formulae (I) to (IV) are each 4 to 12 on the average.

**3.** A liquid softener composition as set forth in claim 1, **characterized in that** the component (A) of the softener base material (2) is a mixture comprising the compounds (V), (VI), (VII) and (VIII), wherein

the weight ratio of the compound (V) to the sum total of the compounds (V), (VI), (VII) and (VIII) ranges from 0.047 to 0.346.
the weight ratio of the compound (VI) to the sum total of the compounds (V), (VI), (VII) and (VIII) ranges from 0.310 to 0.458,
the weight ratio of the compound (VII) to the sum total of the compounds (V), (VI), (VII) and (VIII) ranges from 0.154 to 0.641,
the weight ratio of the compound (VIII) to the sum total of the compounds (V), (VI), (VII) and (VIII) ranges from

0.002 to 0.096,

and the sums of x, y and z in the general formulae (V) to (VIII) are each 3 to 50 on the average, except the mixtures falling under the category of the softener base material (1).

**4.** A liquid softener composition as set forth in claim 1, **characterized in that** the component (A) of the softener base material (2) is a mixture comprising the compounds (V), (VI), (VII) and (VIII) wherein

the weight ratio of the compound (V) to the sum total of the compounds (V), (VI), (VII) and (VIII) ranges from 0.163 to 0.346,
the weight ratio of the compound (VI) to the sum total of the compounds (V), (VI), (VII) and (VIII) ranges from 0.404 to 0.458, .
the weight ratio of the compound (VII) to the sum total of the compounds (V), (VI), (VII) and (VIII) ranges from 0.154 to 0.375,
the weight ratio of the compound (VIII) to the sum total of the compounds (V), (VI), (VII) and (VIII) ranges from 0.017 to 0.096,

and the sums of x, y and z in the general formulae (V) to (VIII) are each 3 to 40 on the average, except the mixtures falling under the category of the softener base material (1).

**5.** A liquid softener composition comprising the softener base material (1) as defined in claim 1 and the component (B) of the softener base material (2) as defined in claim 1 wherein the weight ratio of the softener base material (1) to the component (B) ranges from 2/1 to 1/9.

**6.** A liquid softener composition as set forth in claim 1, 2 or 5, wherein Rs in the' general formulae (I) to (III) are at least one member selected from the group consisting of alkyl group resulting from tallow fatty acid, alkyl resulting from hardened tallow fatty acid, alkyl group resulting from palm stearic acid and alkyl resulting from hardened palm stearic acid.

**7.** A liquid softener composition as set forth in claim 1, 3, 4 or 5, wherein Rs in the general formulae (V) to (VII) are at least one member selected from the group consisting of alkyl group resulting from coconut oil fatty acid, alkyl group resulting from palm kernel oil fatty acid, alkyl group resulting from palm oil fatty acid, alkyl group resulting from palm stearic acid, alkyl group resulting from hardened palm stearic acid, alkyl group resulting from tallow fatty acid and alkyl group resulting from hardened tallow fatty acid.

**8.** A liquid softener composition as set forth in claim 1, 2, 5 or 6, wherein Qs in the general formulas (I) to (IV) are $-CH_2CH_2-$.

**9.** A liquid softener composition as set forth in claim 1, 3, 4, 5 or 7, wherein Qs in the general formulae (V) to (VIII) are $-CH_2CH_2-$.

**10.** A liquid softener composition as set forth in any of claims 1 to 9, wherein the amount of the softener base material (1) and/or (2) is 3 to 40% by weight based on the composition.

**11.** A liquid softener composition as set forth in any of claims 1 to 10, which further contains the following component (C) with the provisoes that the amount of the component (C) is 0.5 to 5% by weight based on the composition, that the weight ratio of the component (C) to the softener base material (1) and/or (2) ranges from 1/100 to 1/2.5 and that the total amount of the softener base material (1) and/or (2) and the component (C) is 3.5 to 45% by weight based on the composition:

[component (C)]
a polyether compound prepared by the addition reaction of a compound having at least 3 active hydrogen atoms with an alkylene oxide having 2 or 3 carbon atoms (wherein the total amount of oxyethylene group moieties is 55% by weight or above based on the component (C)) and having an average molecular weight of 5,000 to 2,000,000, or a derivative thereof.

**12.** A liquid softener composition as set forth in any of claims 1 to 11, which further contains a linear or branched, saturated or unsaturated fatty acid having 8 to 24 carbon atoms as component (D) with the provisoes that the

weight ratio of the component (D) to the softener base material (1) and/or (2) ranges from 0.5/100 to 1/1 and that the total amount of the component (D) and the softener base material (1) and/or (2) is 4 to 50% by weight based on the composition.

## Patentansprüche

1. Flüssige Weichmacherzusammensetzung, **dadurch gekennzeichnet, dass** sie das folgende Weichmachergrundmaterial (1) oder (2) und Wasser umfasst:

**Weichmachergrundmaterial (1):**

eine Mischung, umfassend eine durch die folgende allgemeine Formel (I) dargestellte Verbindung, eine durch die folgende allgemeine Formel (II) dargestellte Verbindung, eine durch die folgende allgemeine Formel (III) dargestellte Verbindung und eine durch die folgende allgemeine Formel (IV) dargestellte Verbindung, unter der Massgabe, dass

das Gewichtsverhältnis der Verbindung (I) zu der Gesamtsumme der Verbindungen (I), (II), (III) und (IV) im Bereich von 0,040 bis 0,327 liegt,

das Gewichtsverhältnis der Verbindung (II) zu der Gesamtsumme der Verbindungen (I), (II), (III) und (IV) im Bereich von 0,298 bis 0,469 liegt,

das Gewichtsverhältnis der Verbindung (III) zu der Gesamtsumme der Verbindungen (I), (II), (III) und (IV) im Bereich von 0,173 bis 0,661 liegt,

das Gewichtsverhältnis der Verbindung (IV) zu der Gesamtsumme der Verbindungen (I), (II), (III) und (IV) im Bereich von 0,001 bis 0,077 liegt,

und dass die Mischung, umfassend die Verbindungen (I), (II), (III) und (IV), einen HLB-Wert nach Griffin im Bereich von 5 bis 12 aufweist:

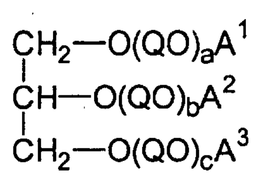

(I)

(wobei

$A^1$, $A^2$ und $A^3$ jeweils eine RCO-Gruppe oder ein Wasserstoffatom darstellen, unter der Massgabe, dass ein Vertreter von $A^1$, $A^2$ und $A^3$ eine RCO-Gruppe darstellt und die anderen Vertreter jeweils ein Wasserstoffatom darstellen, wobei R eine geradkettige oder verzweigte Alkyl- oder Alkenylgruppe mit 7 bis 23 Kohlenstoffatomen darstellt,

Q eine Alkylengruppe mit 2 oder 3 Kohlenstoffatomen oder eine Alkylengruppenmischung, umfassend eine mit 2 Kohlenstoffatomen und eine mit 3 Kohlenstoffatomen, darstellt, und

a, b und c: jeweils eine Zahl von 0 oder mehr darstellen, unter der Massgabe, dass die Summe von a, b und c durchschnittlich 4 bis 18 beträgt),

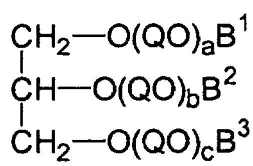

(II)

(wobei

$B^1$, $B^2$ und $B^3$ jeweils eine RCO-Gruppe oder ein Wasserstoffatom darstellen, unter der Massgabe, dass zwei Vertreter von $B^1$, $B^2$ und $B^3$ jeweils eine RCO-Gruppe darstellen und der andere Vertreter ein Wasserstoffatom darstellt, wobei R wie oben definiert ist, und

Q, a, b und c wie oben definiert sind),

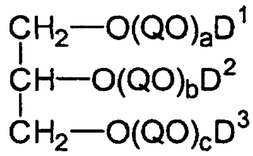

( III )

(wobei

$D^1$, $D^2$ und $D^3$ jeweils eine RCO-Gruppe darstellen, wobei R wie oben definiert ist, und

Q, a, b und c wie oben definiert sind), und

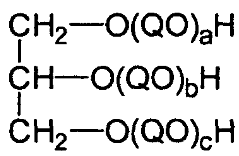

( IV )

(wobei

Q, A, b und c wie oben definiert sind) und

**Weichmachergrundmaterial (2):**
eine Mischung, umfassend die folgenden Komponenten (A) und (B), wobei das Gewichtsverhältnis der Komponente (A) zur Komponente (B) im Bereich von 2:1 bis 1:9 liegt:

**Komponente (A):**

eine Mischung, umfassend eine durch die folgende allgemeine Formel (V) dargestellte Verbindung, eine durch die folgende allgemeine Formel (VI) dargestellte Verbindung, eine durch die folgende allgemeine Formel (VII) dargestellte Verbindung und eine durch die folgende allgemeine Formel (VIII) dargestellte Verbindung,

unter der Massgabe, dass

das Gewichtsverhältnis der Verbindung (V) zu der Gesamtsumme der Verbindungen (V), (VI), (VII) und (VIII) im Bereich von 0,040 bis 0,527 liegt,

das Gewichtsverhältnis der Verbindung (VI) zu der Gesamtsumme der Verbindungen (V), (VI), (VII) und (VIII) im Bereich von 0,133 bis 0,469 liegt,

das Gewichtsverhältnis der Verbindung (VII) zu der Gesamtsumme der Verbindungen (V), (VI), (VII) und (VIII) im Bereich von 0,013 bis 0,661 liegt,

das Gewichtsverhältnis der Verbindung (VIII) zu der Gesamtsumme der Verbindungen (V), (VI), (VII) und (VIII) im Bereich von 0,001 bis 0,417 liegt,

und dass die Mischung, umfassend die Verbindungen (V), (VI), (VII) und (VIII), einen HLB-Wert nach

Griffin im Bereich von 5 bis 15 aufweist, mit Ausnahme der Mischungen, die unter die Kategorie des Weichmachergrundmaterials (1) fallen:

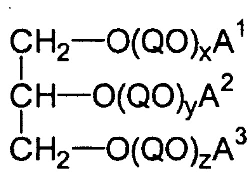 (V)

(wobei

$A^1$, $A^2$, $A^3$ und Q jeweils wie oben definiert sind und

x, y, und z jeweils eine Zahl von 0 oder mehr ist, unter der Massgabe, dass die Summe von x, y und z durchschnittlich im Bereich von 1 bis 50 liegt),

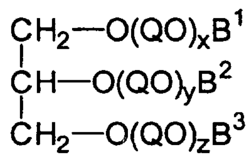 (VI)

(wobei $B^1$, $B^2$, $B^3$, Q, x, y und z jeweils wie oben definiert sind),

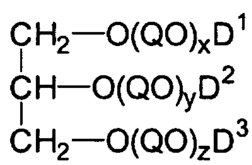 (VII)

(wobei $D^1$, $D^2$, $D^3$, Q, x, y und z jeweils wie oben definiert sind) und

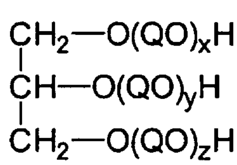 (VIII)

(wobei Q, x, y, und z jeweils wie oben definiert sind), und

**Komponente (B):**
mindestens ein Vertreter, ausgewählt aus den folgenden Komponenten (B-1) und (B-2):

**Komponente (B-1):**
eine durch die folgende allgemeine Formel (X) dargestellte Verbindung:

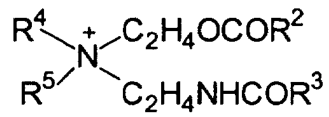 $X^-$ (X)

(wobei

$R^2$ und $R^3$ gleich oder verschieden voneinander sind und eine geradkettige oder verzweigte Alkyl- oder Alkenylgruppe mit 7 bis 23 Kohlenstoffatomen darstellen,

$R^4$ und $R^5$ jeweils eine Alkylgruppe mit 1 bis 4 Kohlenstoffatomen darstellen, und

$X^-$ ein Halogenion oder $R^4SO_4$ darstellt, wobei $R^4$ wie oben definiert ist), und

**Komponente (B-2):**
eine durch die folgende allgemeine Formel (XI) dargestellte Verbindung:

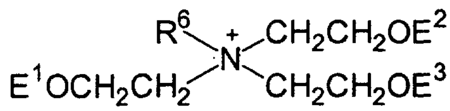 $Y^-$ (XI)

(wobei

$E^1$, $E^2$ und $E^3$ jeweils eine $R^7CO$-Gruppe oder ein Wasserstoffatom darstellen, unter der Massgabe, dass mindestens ein Vertreter von $E^1$, $E^2$ und $E^3$ eine $R^7CO$-Gruppe darstellen, wobei $R^7$ eine geradkettige oder verzweigte Alkyl- oder Alkenylgruppe mit 7 bis 23 Kohlenstoffatomen darstellt,

$R^6$ eine Alkylgruppe mit 1 bis 4 Kohlenstoffatomen darstellt, und

$Y^-$ ein Halogenion oder $R^6SO_4$ darstellt, wobei $R^6$ wie oben definiert ist).

**2.** Weichmacherzusammensetzung gemäss Anspruch 1, die ein Weichmachergrundmaterial (1), umfassend die Verbindungen (I), (II), (III) und (IV) enthält, wobei

das Gewichtsverhältnis der Verbindung (I) zu der Gesamtsumme der Verbindungen (I), (II), (III) und (IV) im Bereich von 0,040 bis 0,317 liegt,

das Gewichtsverhältnis der Verbindung (II) zu der Gesamtsumme der Verbindungen (I), (II), (III) und (IV) im Bereich von 0,298 bis 0,469 liegt,

das Gewichtsverhältnis der Verbindung (III) zu der Gesamtsumme der Verbindungen (I), (II), (III) und (IV) im Bereich von 0,183 bis 0,661 liegt,

das Gewichtsverhältnis der Verbindung (IV) zu der Gesamtsumme der Verbindungen (I), (II), (III) und (IV) im Bereich von 0,001 bis 0,067 liegt,

und die Summe von a, b und c in den allgemeinen Formeln (I) bis (IV) jeweils durchschnittlich 4 bis 12 beträgt.

**3.** Flüssige Weichmacherzusammensetzung gemäss Anspruch 1, **dadurch gekennzeichnet, dass** die Komponente (A) des Weichmachergrundmaterials (2) eine Mischung, umfassend die Verbindungen (V), (VI), (VII) und (VIII) ist, wobei

das Gewichtsverhältnis der Verbindung (V) zu der Gesamtsumme der Verbindungen (V), (VI), (VII) und (VIII) im Bereich von 0,047 bis 0,346 liegt,

das Gewichtsverhältnis der Verbindung (VI) zu der Gesamtsumme der Verbindungen (V), (VI), (VII) und (VIII) im Bereich von 0,310 bis 0,458 liegt,

das Gewichtsverhältnis der Verbindung (VII) zu der Gesamtsumme der Verbindungen (V), (VI), (VII) und (VIII) im Bereich von 0,154 bis 0,641 liegt,

das Gewichtsverhältnis der Verbindung (VIII) zu der Gesamtsumme der Verbindungen (V), (VI), (VII) und (VIII) im Bereich von 0,002 bis 0,096 liegt,

und die Summe von x, y und z in den allgemeinen Formeln (V) bis (VIII) jeweils durchschnittlich 3 bis 50 beträgt, ausgenommen die Mischungen, die unter die Kategorie des Weichmachergrundmaterials (1) fallen.

**4.** Flüssige Weichmacherzusammensetzung gemäss Anspruch 1, **dadurch gekennzeichnet, dass** die Komponente (A) des Weichmachergrundmaterials (2) eine Mischung, umfassend die Verbindungen (V), (VI), (VII) und (VIII) ist, wobei

das Gewichtsverhältnis der Verbindung (V) zu der Gesamtsumme der Verbindungen (V), (VI), (VII) und (VIII) im Bereich von 0,163 bis 0,346 liegt,

das Gewichtsverhältnis der Verbindung (VI) zu der Gesamtsumme der Verbindungen (V), (VI), (VII) und (VIII) im Bereich von 0,404 bis 0,458 liegt,

das Gewichtsverhältnis der Verbindung (VII) zu der Gesamtsumme der Verbindungen (V), (VI), (VII) und (VIII) im Bereich von 0,154 bis 0,375 liegt,

das Gewichtsverhältnis der Verbindung (VIII) zu der Gesamtsumme der Verbindungen (V), (VI), (VII) und (VIII) im Bereich von 0,017 bis 0,096 liegt,

und die Summe von x, y und z in den allgemeinen Formeln (V) bis (VIII) jeweils durchschnittlich 3 bis 40 beträgt, ausgenommen die Mischungen, die unter die Kategorie des Weichmachergrundmaterials (1) fallen.

**5.** Flüssige Weichmacherzusammensetzung, umfassend das in Anspruch 1 definierte Weichmachergrundmaterial (1) und die Komponente (B) des in Anspruch 1 definierten Weichmachergrundmaterials (2), wobei das Gewichtsverhältnis des Weichmachergrundmaterials (1) zu der Komponente (B) im Bereich von 2:1 bis 1:9 liegt.

**6.** Flüssige Weichmacherzusammensetzung gemäss Anspruch 1, 2 oder 5, wobei R in den allgemeinen Formeln (I) bis (III) mindestens ein Vertreter, ausgewählt aus der Gruppe bestehend aus einer Alkylgruppe von Talg-Fettsäure, Alkyl von gehärteter Talg-Fettsäure, einer Alkylgruppe von Palmstearinsäure und einer Alkylgruppe von gehärteter Palmstearinsäure ist.

**7.** Flüssige Weichmacherzusammensetzung gemäss Anspruch 1, 3, 4 oder 5, wobei R in den allgemeinen Formeln (V) bis (VII) mindestens ein Vertreter, ausgewählt aus der Gruppe bestehend aus einer Alkylgruppe der Kokosnussöl-Fettsäure, einer Alkylgruppe der Palmkernöl-Fettsäure, einer Alkylgruppe der Palmöl-Fettsäure, einer Alkylgruppe der Palmstearinsäure, einer Alkylgruppe der gehärteten Palmstearinsäure, einer Alkylgruppe der Talg-Fettsäure und einer Alkylgruppe der gehärteten Talg-Fettsäure ist.

**8.** Flüssige Weichmacherzusammensetzung nach Anspruch 1, 2, 5 oder 6, wobei Q in den allgemeinen Formeln (I) bis (IV) $-CH_2CH_2-$ ist.

**9.** Flüssige Weichmacherzusammensetzung nach Anspruch 1, 3, 4, 5 oder 7, wobei Q in den allgemeinen Formeln (V) bis (VIII) $-CH_2CH_2-$ ist.

**10.** Flüssige Weichmacherzusammensetzung gemäss einem der Ansprüche 1 bis 9, wobei die Menge des Weichmachergrundmaterials (1) und/oder (2) 3 bis 40 Gew.% in bezug auf die Zusammensetzung beträgt.

**11.** Flüssige Weichmacherzusammensetzung nach einem der Ansprüche 1 bis 10, die weiterhin die folgende Komponente (C) enthält, unter den Massgaben, dass die Menge der Komponente (C) 0,5 bis 5 Gew.% in bezug auf die Zusammensetzung beträgt, dass das Gewichtsverhältnis der Komponente (C) zu dem Weichmachergrundmaterial (1) und/oder (2) im Bereich von 1:100 bis 1:2,5 liegt, und dass die Gesamtmenge des Weichmachergrundmaterials (1) und/oder (2) und der Komponente (C) 3,5 bis 45 Gew.% in bezug auf die Zusammensetzung beträgt:

**Komponente (C):**

eine Polyetherverbindung, hergestellt durch Additionsreaktion einer Verbindung mit mindestens 3 aktiven Wasserstoffatomen mit einem Alkylenoxid mit 2 oder 3 Kohlenstoffatomen (wobei die Gesamtmenge an Oxyethylengruppen 55 Gew.% oder mehr in bezug auf die Komponente (C) beträgt) und mit einem durchschnittlichen Molekulargewicht von 5.000 bis 2.000.000, oder ein Derivat hiervon.

**12.** Flüssige Weichmacherzusammensetzung gemäss einem der Ansprüche 1 bis 11, die weiterhin eine geradkettige oder verzweigte, gesättigte oder ungesättigte Säure mit 8 bis 24 Kohlenstoffatomen als Komponente (D) enthält, unter den Massgaben, dass das Gewichtsverhältnis der Komponente (D) zu dem Weichmachergrundmaterial (1) und/oder (2) im Bereich von 0,5:100 bis 1:1 liegt, und dass die Gesamtmenge der Komponente (D) und des Weichmachergrundmaterials (1) und/oder (2) 4 bis 50 Gew.% in bezug auf die Zusammensetzung beträgt.

## Revendications

**1.** Composition adoucissante liquide, **caractérisée en ce qu'**elle comprend la substance de base adoucissante (1) ou (2) suivante et de l'eau :

[substance de base adoucissante (1)]
un mélange comprenant un composé représenté par la formule générale (I) suivante, un composé représenté par la formule générale (II) suivante, un composé représenté par la formule générale (III) suivante et un composé représenté par la formule générale (IV) suivante, à condition que

le rapport en poids du composé (I) à la somme totale des composés (I), (II), (III) et (IV) se situe dans la gamme de 0,040 à 0,327,
le rapport en poids du composé (II) à la somme totale des composés (I), (II), (III) et (IV) se situe dans la gamme de 0,298 à 0,469,
le rapport en poids du composé (III) à la somme totale des composés (I), (II), (III) et (IV) se situe dans la gamme de 0,173 à 0,661, et
le rapport en poids du composé (IV) à la somme totale des composés (I), (II), (III) et (IV) se situe dans la gamme de 0,001 à 0,077,

et que le mélange comprenant les composés (I), (II), (III) et (IV) ait un indice HLB de Griffin se situant dans la gamme de 5 à 12 :

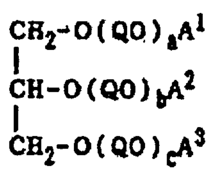

(I)

[dans laquelle

$A^1$, $A^2$ et $A^3$ : chacun un groupe RCO ou un atome d'hydrogène, à condition que l'un de $A^1$, $A^2$ et $A^3$ soit un groupe RCO et les autres soient chacun un atome d'hydrogène, où R représente un groupe alkyle ou alcényle linéaire ou ramifié comportant 7 à 23 atomes de carbone,
Q : un groupe alkylène comportant 2 ou 3 atomes de carbone ou un mélange de groupes alkylène comprenant un groupe alkylène comportant 2 atomes de carbone et un groupe alkylène comportant 3 atomes de carbone, et
a, b et c : chacun un nombre de 0 ou plus, à condition que la somme de a, b et c soit de 4 à 18 en moyenne],

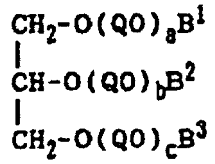

(II)

[dans laquelle

B[1], B[2] et B[3] : chacun un groupe RCO ou un atome d'hydrogène, à condition que deux de B[1], B[2] et B[3] soient un groupe RCO et l'autre soit un atome d'hydrogène, où R est tel que défini ci-dessus, et

Q, a, b et c : chacun tels que définis ci-dessus],

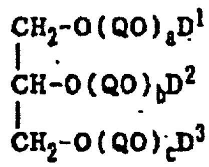

(III)

[dans laquelle

D[1], D[2] et D[3] : chacun un groupe RCO, où R est tel que défini ci-dessus, et

Q, a, b et c : chacun tels que définis ci-dessus], et

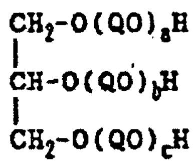

(IV)

[dans laquelle

Q, a, b et c : chacun tels que définis ci-dessus], et

[substance de base adoucissante (2)

un mélange comprenant les composants (A) et (B) suivants, dans lequel le rapport en poids du composant (A) au composant (B) se situe dans la gamme de 2/1 à 1/9 :

<composant (A)>

un mélange comprenant un composé représenté par la formule générale (V) suivante, un composé représenté par la formule générale (VI) suivante, un composé représenté par la formule générale (VII) suivante, un composé représenté par la formule générale (VIII) suivante, à condition que

le rapport en poids du composé (V) à la somme totale des composés (V), (VI), (VII) et (VIII) se situe dans la gamme de 0,040 à 0,527,
le rapport en poids du composé (VI) à la somme totale des composés (V), (VI), (VII) et (VIII) se situe dans la gamme de 0,133 à 0,469,
le rapport en poids du composé (VII) à la somme totale des composés (V), (VI), (VII) et (VIII) se situe dans la gamme de 0,013 à 0,661, et
le rapport en poids du composé (VIII) à la somme totale des composés (V), (VI), (VII) et (VIII) se situe dans la gamme de 0,001 à 0,417,

et que le mélange comprenant les composés (V), (VI), (VII) et (VIII) ait un indice HLB de Griffin se situant

dans la gamme de 5 à 15, excepté les mélanges entrant dans la catégorie de la substance de base adoucissante (1) :

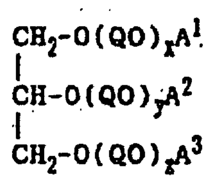

(V)

[dans laquelle

$A^1$, $A^2$, $A^3$ et Q : chacun tels que définis ci-dessus, et

x, y et z : chacun un nombre entier de 0 ou plus, à condition que la somme de x, y et z soit de 1 à 50 en moyenne],

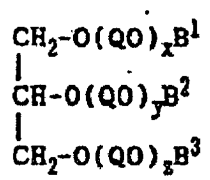

(VI)

[dans laquelle

$B^1$, $B^2$, $B^3$, Q, x, y et z : chacun tels que définis ci-dessus]

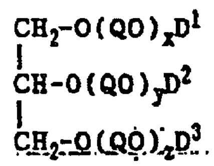

(VII)

[dans laquelle

$D^1$, $D^2$, $D^3$, Q, x, y et z : chacun tels que définis ci-dessus]

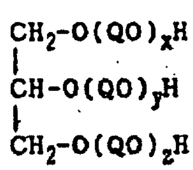

(VIII)

[dans laquelle

Q, x, y et z : chacun tels que définis ci-dessus], et

<composant (B)>

au moins un élément choisi parmi les composants (B-1) et (B-2) suivants :

<<composant (B-1)>>

un composé représenté par la formule générale (X) suivante :

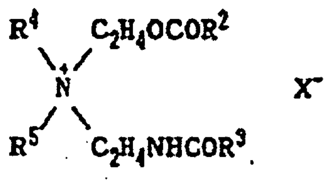

(X)

[dans laquelle

$R^2$ et $R^3$ : identiques ou différents l'un de l'autre, un groupe alkyle ou alcényle linéaire ou ramifié comportant 7 à 23 atomes de carbone,
$R^4$ et $R^5$ : chacun un groupe alkyle comportant 1 à 4 atomes de carbone, et
$X^-$ : un ion halogène ou $R^4SO_4$, où $R^4$ est tel que défini ci-dessus], et

<<composant (B-2)>>
un composé représenté par la formule générale (XI) suivante :

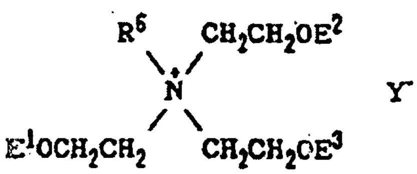

(XI)

[dans laquelle

$E^1$, $E^2$ et $E^3$ : chacun un groupe $R^7CO$ ou un atome d'hydrogène, à condition que l'un au moins de $E^1$, $E^2$ et $E^3$ soit un groupe $R^7CO$, où $R^7$ représente un groupe alkyle ou alcényle linéaire ou ramifié comportant 7 à 23 atomes de carbone,
$R^6$ : un groupe alkyle comportant 1 à 4 atomes de carbone, et
$Y^-$ : un ion halogène ou $R^6SO_4$, où $R^6$ est tel que défini ci-dessus].

**2.** Composition adoucissante liquide selon la revendication 1, qui contient une substance de base adoucissante (1) comprenant les composés (I), (II), (III) et (IV), dans laquelle

le rapport en poids du composé (I) à la somme totale des composés (I), (II), (III) et (IV) se situe dans la gamme de 0,040 à 0,317,
le rapport en poids du composé (II) à la somme totale des composés (I), (II), (III) et (IV) se situe dans la gamme de 0,298 à 0,469,
le rapport en poids du composé (III) à la somme totale des composés (I), (II), (III) et (IV) se situe dans la gamme de 0,183 à 0,661,
le rapport en poids du composé (IV) à la somme totale des composés (I), (II), (III) et (IV) se situe dans la gamme de 0,001 à 0,067,

et les sommes de a, b et c dans les formules générales (I) à (IV) sont chacune de 4 à 12 en moyenne.

**3.** Composition adoucissante liquide selon la revendication 1, **caractérisée en ce que** le composant (A) de la substance de base adoucissante (2) est un mélange comprenant les composés (V), (VI), (VII) et (VIII), dans laquelle

le rapport en poids du composé (V) à la somme totale des composés (V), (VI), (VII) et (VIII) se situe dans la gamme de 0,047 à 0,346,
le rapport en poids du composé (VI) à la somme totale des composés (V), (VI), (VII) et (VIII) se situe dans la gamme de 0,310 à 0,458,

le rapport en poids du composé (VII) à la somme totale des composés (V), (VI), (VII) et (VIII) se situe dans la gamme de 0,154 à 0,641,
le rapport en poids du composé (VIII) à la somme totale des composés (V), (VI), (VII) et (VIII) se situe dans la gamme de 0,002 à 0,096,

et les sommes de x, y et z dans les formules générales (V) à (VIII) sont chacune de 3 à 50 en moyenne, excepté les mélanges entrant dans la catégorie de la substance de base adoucissante (1).

4. Composition adoucissante liquide selon la revendication 1, **caractérisée en ce que** le composant (A) de la substance de base adoucissante (2) est un mélange comprenant les composés (V), (VI), (VII) et (VIII), dans laquelle

le rapport en poids du composé (V) à la somme totale des composés (V), (VI), (VII) et (VIII) se situe dans la gamme de 0,163 à 0,346,
le rapport en poids du composé (VI) à la somme totale des composés (V), (VI), (VII) et (VIII) se situe dans la gamme de 0,404 à 0,458,
le rapport en poids du composé (VII) à la somme totale des composés (V), (VI), (VII) et (VIII) se situe dans la gamme de 0,154 à 0,375,
le rapport en poids du composé (VIII) à la somme totale des composés (V), (VI), (VII) et (VIII) se situe dans la gamme de 0,017 à 0,096,

et les sommes de x, y et z dans les formules générales (V) à (VIII) sont chacune de 3 à 40 en moyenne, excepté les mélanges entrant dans la catégorie de la substance de base adoucissante (1).

5. Composition adoucissante liquide comprenant la substance de base adoucissante (1) telle que définie dans la revendication 1 et le composant (B) de la substance de base adoucissante (2) telle que définie dans la revendication 1, dans laquelle le rapport en poids de la substance de base adoucissante (1) au composant (B) se situe dans la gamme de 2/1 à 1/9.

6. Composition adoucissante liquide selon la revendication 1, 2 ou 5, dans laquelle les R dans les formules générales (I) à (III) sont au moins un élément choisi dans le groupe constitué par un groupe alkyle provenant d'un acide gras de suif, un alkyle provenant d'un acide gras de suif durci, un groupe alkyle provenant d'un acide stéarique de palme et un alkyle provenant d'un acide stéarique de palme durci.

7. Composition adoucissante liquide selon la revendication 1, 3, 4 ou 5, dans laquelle les R dans les formules générales (V) à (VII) sont au moins un élément choisi dans le groupe constitué par un groupe alkyle provenant d'un acide gras d'huile de noix de coco, un groupe alkyle provenant d'un acide gras d'huile d'amande de palme, un groupe alkyle provenant d'un acide gras d'huile de palme, un groupe alkyle provenant d'un acide stéarique de palme, un groupe alkyle provenant d'un acide stéarique de palme durci, un groupe alkyle provenant d'un acide gras de suif, un groupe alkyle provenant d'un acide gras de suif durci.

8. Composition adoucissante liquide selon la revendication 1, 2, 5 ou 6, dans laquelle les Q dans les formules générales (I) à (IV) sont $-CH_2CH_2-$.

9. Composition adoucissante liquide selon la revendication 1, 3, 4, 5 ou 7, dans laquelle les Q dans les formules générales (V) à (VIII) sont $-CH_2CH_2-$.

10. Composition adoucissante liquide selon l'une quelconque des revendications 1 à 9, dans laquelle la quantité de la substance de base adoucissante (1) et/ou (2) est de 3 à 40 % en poids, par rapport à la composition.

11. Composition adoucissante liquide selon l'une quelconque des revendications 1 à 10, qui contient en outre le composant (C) suivant, à condition que la quantité du composant (C) soit de 0,5 à 5 % en poids par rapport à la composition, que le rapport en poids du composant (C) à la substance de base adoucissante (1) et/ou (2) se situe dans la gamme de 1/100 à 1/2,5 et que la quantité totale de la substance de base adoucissante (1) et/ou (2) et du composant (C) soit de 3,5 à 45 % en poids, par rapport à la composition :

[composant (C)]
un composé polyéther préparé par une réaction d'addition d'un composé comprenant au moins trois atomes d'hydrogène actifs avec un oxyde d'alkylène comportant 2 ou 3 atomes de carbone [où la quantité

totale de groupements oxyéthylène est de 55 % en poids

ou plus, par rapport au composant (C)] et ayant un poids moléculaire moyen de 5 000 à 2 000 000, ou un dérivé de celui-ci.

**12.** Composition adoucissante liquide selon l'une quelconque des revendications 1 à 11, qui contient en outre un acide gras linéaire ou ramifié, saturé ou insaturé, comportant 8 à 24 atomes de carbone en tant que composant (D), à condition que le rapport en poids du composant (D) à la substance de base adoucissante (1) et/ou (2) se situe dans la gamme de 0,5/100 à 1/1 et que la quantité totale du composant (D) et de la substance de base adoucissante (1) et/ou (2) soit de 4 à 50 % en poids, par rapport à la composition.